(19) **Europäisches Patentamt / European Patent Office / Office européen des brevets**

(11) **EP 3 348 287 A1**

(12) # EUROPEAN PATENT APPLICATION
published in accordance with Art. 153(4) EPC

(43) Date of publication:
**18.07.2018 Bulletin 2018/29**

(51) Int Cl.:
**A61L 31/02** *(2006.01)* **A61B 17/22** *(2006.01)*

(21) Application number: **16850377.9**

(22) Date of filing: **29.09.2016**

(86) International application number:
**PCT/CN2016/100804**

(87) International publication number:
**WO 2017/054753 (06.04.2017 Gazette 2017/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.09.2015 CN 201510631394**

(71) Applicant: **Shanghai Clinical Engine Technology Development Co., Ltd.**
**Shanghai 200439 (CN)**

(72) Inventor: **SUN, Yinghao**
**Shanghai 200433 (CN)**

(74) Representative: **Prüfer & Partner mbB**
**Patentanwälte · Rechtsanwälte**
**Sohnckestraße 12**
**81479 München (DE)**

(54) **USE OF MAGNETIC MATERIALS IN REMOVING CALCULUS**

(57) The invention relates to a use of a magnetic material in stone removal, nanoparticles, a preparation method thereof and a stone removing device. Wherein, the magnetic material constitutes a nanoparticle core and a surface modifier monomer is attached to the nanoparticle core by an initiator and / or a crosslinking agent to form a nanoparticle shell. The prepared nanoparticles can surround stones in ureter, thereby, small stones remaining in body can be removed quickly without damage from the body under action of external magnetic field, that is, the stone can be drawn and moved without injuring ureteral wall, and the nanoparticles are placed conveniently without shift.

EP 3 348 287 A1

**Description**

**Technical field**

The present invention relates to a use of a magnetic material in removal of stones, nano-particles, a method for preparing the same, includes a device comprising nano-particles for removal of stones.

**Background Art**

Urinary calculi/stone (urolithiasis) have an incidence as high as 5% -10%, and can be found in any part of kidney, bladder, ureter and urethra, in which stones in kidney and ureter are common. It is found in clinical observation that calcium-containing stones are the most common types of urinary stones, that is, about 70% to 80% of all urinary stones. At present, there are only a few cases of calcium-containing stones which pathological causes have been clearly revealed, while the causes for most of calcium-containing stones are not yet clear. According to chemical composition, stones can be divided into four categories: calcium-containing stones, infection induced stones, uric acid stones and cystine stones. Calcium-containing stones can be divided into following types: simple calcium oxalate, calcium oxalate with calcium phosphate, calcium oxalate with a small amount of uric acid; the main components of infection induced stones are ammonium magnesium phosphate and hydroxyapatite; uric acid stones can be divided into following types: uric acid, uric acid amine, or those containing a small amount of calcium oxalate in addition to the above ingredients; cystine stones can be divided into following types: simple cystine, or cystine with a small amount of calcium oxalate.

Soft/hard ureteroscopic lithotripsy is performed through natural channel of the human body, has the advantages of small trauma and definite lithothriptic effect, and is currently the main treatment means for most of ureteral stones and kidney stones. However, the current soft/hard ureteroscopic lithotripsy also has some deficiencies: 1) upper ureteral stones and fragments of stones in ureter may be easily brought back to kidney by the infused water or the recoil force of lithotripsy tools; 2) there is lack of a fast, safe and effective method to take out residual debris of stones in ureteral lumen and kidney calices. It is an an important mean to prevent ureteral stones from being recoiled back to kidney that a tool is used to block the ureter above the ureteral stones. At present, there are also some ureteral occluders in clinical practice, and such kind of stones blocking tools are also commonly used to remove stones. However, these ureteral occluders still have some shortcomings in actual use. Stone baskets (such as various stone baskets described in Patent Publication No. JP2009536081A, DE19904569A1, WO2004056275A1, WO2011123274A1 are designed with a net bag) are the most commonly used stone interception and removal tools, which cross over stone and then are opened to form a net so as to prevent the upward drift of stone debris, and at the same time, the stone baskets are also used as stone removal tools to net and take out small stone debris. However, the amount of stone removed by the stone basket every time is limited, so that multiple feeds of ureteroscope are needed, while repeated injection of water and feeds of ureteroscope would increase the risk of residual debris drift; in addition, the stone basket cannot completely seal the ureteral lumen, and there are still a chance that stones escape from the net; moreover, the edges of stones in stone basket may be easily squeezed out from basket holes, and thus may easily scratch the ureter wall when the stones are dragged and removed, thereby causing complications in severe cases.

In addition, CN105283140A describes a kit of a cross-linked gel for encapsulating urethral stones and/or debris of urethral stones. However, when a gel-containing magnetic reagent (mock) is added to an aqueous solution containing stones, the cationic polymer is dispersed substantially in the form of dissolving in a solvent rather than aggregates to the periphery of stones due to the principle of similar compatibility, which is not sufficient to magnetize stones and remove stones.

In summary, there is an urgent need for a material and method for removal of stone in urinary system that can collect stones, facilitate the stone taking, do not damage the ureteral wall when the stones are dragged, can be conveniently placed and are not easy to cause shift of stone.

**Contents of the Invention**

The present invention is aimed to solve the problem of residual stone debris and difficult removal thereof in the conventional soft/hard ureteroscopic lithotripsy. Therefore, a first object of the present invention is to provide a use of a magnetic material in removal of stones; a second object of the present invention is to provide a magnetic material capable of safely and efficiently removing urinary stones located in the kidney, ureter, etc., wherein the magnetic material is a magnetic nano-material; a third object of the present invention is to provide a method for preparing the magnetic material in different morphological structures, wherein the magnetic material is a magnetic nano-material; a fourth object of the present invention is to provide a use of the magnetic material in combination with a self-made magnetic probe rod system in a urinary stones surgery,wherein the magnetic material is a magnetic nano-material; a fifth object of the present invention is to provide a use of the magnetic material in manufacture of an article for removal of stones in urinary system, wherein the magnetic material is a magnetic nano-material.

A first aspect of the present invention provides a solution 1, that is, a use of a magnetic material in removal of stones, wherein the magnetic material magnetizes the stones by physical adsorption, chemical bonding or the like, and removes the stones by the action of a non-contact magnetic field.

2. The use according to the embodiment 1, wherein the magnetic material comprises the following components: a preparation containing a magnetic metal element or a compound thereof; and materials capable of binding to calcium salt.

3. The use according to the embodiment 2, wherein the preparation containing the magnetic metal element or compound thereof and the material capable of binding to calcium salt form a structure that may be a clad structure or a core-shell structure, for example, the materials are completely or partially coated on the surface of the preparation; or form a modified structure in which the materials are bonded to the surface of the preparation by absorption; or form a complex structure in which the preparation and the materials form a physical mixture; or form a composite structure of the aforementioned structures.

4. The use according to the embodiment 2, wherein the preparation containing the magnetic metal element or compound thereof is in nano-scale or in micro-scale.

5. The use according to the embodiment 2, wherein the materials capable of binding to calcium salt are surfactant or polymer compounds.

6. The use according to embodiment 2, wherein the materials capable of binding to calcium salt are macromolecular compounds.

7. The use according to the embodiment 1, wherein the magnetic material includes carboxyl, amido, amino, mercapto, hydroxyl, carbonyl, ether group, amine group, ester group, carbamate group, carbamido or quaternary amine group, sulfonic acid group, sulfhydryl, phosphine group or conjugate acid or base thereof, epoxy group, chlorine group, sulfate group, phosphinic acid, sulfinic acid, carboxylic anhydride group, hydrosilyl group, amine group and moiety of any combination thereof, aldehyde group, unsaturated double bond, phosphoric acid group, halogen group, N-succinimido, maleimido, ethylenediaminetriacetic acid alkyl group, polyethylene glycol, polyamino acid or glycan, preferably carboxyl, amido, mercapto, carbamate group, carbamido, sulfonic acid group, phosphino conjugate acid, phosphino basic group, sulfate group, phosphinic acid, sulfinic acid, N-succinimido, maleimido, polyamino acid.

8. The use according to the embodiment 1, wherein the magnetic material is in the shape of bar, line, band, sheet, tube, pomegranate, cube, three-dimensional flower, petal, chestnut, four-pointed star, shuttle, rice grain, sea urchin, chain ball, rugby ball, string of beads, snowflake, ellipsoid, sphere, regular tetrahedron, regular hexahedron, regular octahedron, quasi-sphere, popcorn, cross, strip, rod, cone, disc, branch, web, simple cubic, body-centered cubic, face-centered cubic, simple tetragon, body-centered tetragon, simple orthogon, body-centered orthogon, single-face-centered orthogon, multi-shell, laminar, preferably the shape of sphere, quasi-sphere, pomegranate, chestnut, sea urchin, chain ball, string of beads.

9. The use according to the embodiment 1, wherein the magnetic material includes a magnetic fluid, a magnetic liposome, a magnetic microcapsule, a magnetic microsphere, a magnetic emulsion, a magnetic nanoparticle, a magnetic nanotube, a magnetic nanowire, a magnetic nanorod, a magnetic nanoribbons, preferably a magnetic fluid, a magnetic liposome, a magnetic microsphere, a magnetic nanotube.

10. The use according to the embodiment 9, wherein the magnetic liposome includes a magnetic liposome which surface is modified to carry a functional group as described in the embodiment 7.

11. The use according to the embodiment 9, wherein the type of the magnetic liposome includes a single layer, a multi-layer, a multi-vesicle, and the preparation method of the magnetic liposome includes preferably a film dispersion method and an ultrasonic dispersion method.

12. The use according to the embodiment 9, wherein the magnetic fluid is a stable suspension liquid composed of magnetic particles, a carrier liquid (mineral oil, silicone oil, etc.) and a surfactant, the magnetic particles that can be used for removal of stones comprise magnetic nanoparticles which surface is modified with the functional group according to the embodiment 7, and also comprise a surfactant with the functional group as described in the embodiment 7.

13. The use according to the embodiment 9, wherein the preparation method for the the magnetic fluid includes a chemical co-precipitation method, a sol-gel method, a hydrothermal synthesis method, a microemulsion method, a phase transfer method, preferably a co-precipitation method, a sol-gel method, a hydrothermal synthesis method.

14. The use according to the embodiment 9, wherein the magnetic microsphere is characterized by a surface modified with the functional group as described in the embodiment 7.

15. The use according to the embodiment 9, wherein the preparation method for the magnetic microsphere includes an emulsion volatilization method, a solvent replacement method and a salting-out method.

16. The use according to the embodiment 1, the magnetic nanotube comprises a magnetic nanotube in which a magnetic material is filled in the tube and also a magnetic nanotube in which a magnetic material covers outside the tube, the surface of which has the functional group described in the embodiment 7, and the functional group may be derived from the magnetic material covered on surface or from the nanotube itself.

17. The use according to the embodiment 1, wherein the preparation method for the magnetic nanotube includes a chemical vapor deposition method, a co-precipitation method, a dip-pyrolysis method, an electroless plating method and a self-assembly method, and the preferred method is a co-precipitation method.

18. The use according to the embodiment 1, the magnetic nanoparticle is surface-modified or covered with the functional group as described in the embodiment 7.

19. The use according to the embodiment 1, wherein the magnetic material constitutes a nanoparticle core; and the nanoparticle core is modified in-situ with a surface modifier monomer by using an initiator and / or a crosslinking agent to form a nanoparticle shell.

20. The use according to the embodiment 19, wherein the nanoparticle core has a diameter of 2-50 nm, and a weight percentage of 30-95% relative to the whole weight of the nanoparticle, and its magnetic material includes a compound of $Fe^{3+}$, $Fe^{2+}$, $Mn^{2+}$ or $Ni^{2+}$, or a metal element selected from iron, nickel, copper, cobalt, platinum, gold, europium, gadolinium, dysprosium, terbium, or a composite or oxide of the aforementioned metals, or any one of the above items or a combination of two or more of the above items, preferably a compound of $Fe^{3+}$, $Fe^{2+}$, $Mn^{2+}$ or $Ni^{2+}$, more preferably $Fe^{3+}$ and $Fe^{2+}$ in a ratio of 15% to 85%, preferably 1: 2.5 to 1.5: 1.

21. The use according to the embodiment 19, wherein the mutual forces for surrounding and crosslinking between the nanoparticle and stone include van der Waals force, hydrophobic interaction, adsorption and surface deposition that form surrounding interactions; a chemical bond formed between carboxyl-stone, including a hydrogen bond, an ester bond, an amide bond and other covalent bonds; physical and chemical inter-chain entanglements between chains and chemical crosslinking between chains.

22. The use according to any one of the embodiments 19-22, wherein the surface modifier includes a hydrophilic surface modifier with function response, a hydrophobic surface modifier with function response, a photosensitive surface modifier with function response, a thermosensitive surface modifier with function response or a pH sensitive surface modifier with function response, wherein the hydrophilic surface modifier includes acrylic acid, methacrylic acid, isobutyl acrylamide or poly N-substituted isopropylacrylamide; the hydrophobic surface modifier includes olefins, preferably polystyrene, polyethylene or oleic acid; the photosensitive surface modifier is selected from the group consisting of azos and quinolines and benzophenones (PVBP), preferably ethylene benzophenone; the thermosensitive surface modifier is selected from the group consisting of amphiphilic polymers with amide bond, preferably polyacrylamide or poly N-substituted isopropylacrylamide; the pH-sensitive surface modifier is selected from the group consisting of polymers with carboxyl group and quaternary ammonium salt, preferably a polyacrylic acid, dimethylaminoethyl ester and dimethylaminopropyl methacrylate; the shell accounts for 2-40% by weight of the nano-particle, preferably the particle is of a shape of sphere, rod or diamond.

23. The use according to any one of the embodiments 19 to 22, wherein the crosslinking agent includes 3-(methacryloyloxy)propyltriethoxysilane, divinylbenzene, diisocyanate or N,N-methylenebisacrylamide, and the initiator includes 3-chloropropionic acid, CuCl, 4,4'-dinonyl-2,2-bipyridine or potassium persulfate.

24. The use according to any one of the embodiments 19-22, wherein the preparation method for the nanoparticle includes the steps of:

a) preparation of the nanoparticle core using the magnetic material;
b) forming the nanoparticle by in situ linking the surface modifier monomer to the nanoparticle core by the initiator and / or crosslinking agent to form the nanoparticle shell.

25. The use according to the embodiment 24, wherein the magnetic material includes a compound of $Fe^{3+}$, $Fe^{2+}$, $Mn^{2+}$ or $Ni^{2+}$, or a metal element selected from iron, nickel, copper, cobalt, platinum, gold, europium, gadolinium, dysprosium, terbium, or a composite or oxide of the aforementioned metals, or any one of the above items or a combination of two or more of the above items, preferably $Fe_3O_4$, $MnFe_2O_4$, $\gamma$-$Fe_2O_3$ or other nanoscale-sized ferrite particles, more preferably $FeCl_3 \cdot 6H_2O$ and $FeCl_2 \cdot 4H_2O$ in a molar ratio of 15% to 85%, preferably 1:2.5 to 1.5:1, and is prepared by the following steps:

dissolving a proportion of the metal salt-containing material in water;
feeding nitrogen to expel oxygen in the solution;
adding a catalyst at a room temperature of 10-40°C, preferably 30°C to adjust the pH to 7-12, preferably 10;
keeping agitation for 10-60 minutes; and
reacting under condition of 40-100°C, preferably 70°C water bath, for 20-40 minutes, then separating with a magnet and drying to obtain the magnetic nanoparticle core.

26. The use according to the embodiment 25, wherein when aqueous ammonia is used as the catalyst for preparing the nanoparticle, the method for adding aqueous ammonia is a continuous dropping method with assistance of an electronic pump at a speed of 20-100 drops / minute, preferably 40-60 drops / minute; and when the magnetic material is a liquid monomer material, the liquid monomer is added dropwise in continuous manner with assistance of an electronic pump, and the reaction agitation is at a speed of 100-1000 revolutions / minute, preferably 500-700 revolutions / minute.

27. The use according to any one of the embodiments 24-27, wherein said use further includes performing hydrophobic surface modification on the obtained nanoparticle core, comprising the steps of:

dispersing the prepared nanoparticle core in an aqueous solution and added with a xylene solution of 3-chloropropionic acid, polystyrene, CuCl and 4,4'-dinonyl-2,2-dipyridine, and the molar ration between the above-mentioned nanoparticle core and the reaction solution is 1:1;
reacting the above mixture at 130°C with continuous agitation for 15-30h, preferably for 24 hours; and
collecting the nanoparticle with a magnet and washing repeatedly with toluene to obtain a hydrophobic polystyrene-coated magnetic nanoparticle.

28. The use according to any one of the embodiments 24-27, wherein said use further includes performing hydrophilic surface modification on the obtained nanoparticle core comprising the steps of:

dispersing the nanoparticle core in xylene, and adding a silane coupling agent, wherein the ratio of the added nanoparticles, xylene and silane coupling agent is 95: 5;
reacting under protection of nitrogen atmosphere at a temperature of 20 to 100 °C, preferably 80 °C for 2 to 5 hours, preferably 3 hours;
washing with an alcohol solvent and drying for 12h, dispersing in an aqueous solution under ultrasonic condition, adding with potassium persulfate;
reacting under protection of nitrogen atmosphere at 40-80 °C for 10 minutes, then adding with acrylic acid and continuously reacting at 40-80 °C for 1 hour, preferably reacting at 70 °C; and
separating by a magnet, washing and drying to prepare and obtain a polyacrylic acid-modified hydrophilic nanoparticle.

29. The use according to any one of the embodiments 24-27, wherein said use further includes performing a photosensitive, thermosensitive or pH-sensitive surface modification based on the resulting nanoparticle core or hydrophilic surface, or a hydrophilic, hydrophobic, photosensitive, thermosensitive and pH-sensitive co-modification based on the resulting nanoparticle core, wherein the re-modification on the hydrophilic surface includes the steps of: dissolving and dispersing the polyacrylic acid-modified magnetic nanoparticles in an alcoholic solvent, adding with a photosensitive monomer such as ethylene benzophenone, a thermosensitive monomer such as N-isopropylacrylamide, or a pH-sensitive monomer such as dimethylaminopropyl methacrylate or a blend monomer of acrylic acid and styrene, keeping reaction at 40-80 °C for 1 h, preferably at a reaction temperature of 70 °C; and separating with a magnet, washing and drying to obtain a photosensitive, thermosensitive or pH sensitive functional monomer-modified magnetic nanoparticles, respectively.

30. The use according to the embodiment 1, wherein the stone comprise urinary system stones, such as kidney stones, ureteral stones and bladder stones, human biliary system stones, and stone-like particles in other organs.

31. The use according to the embodiment 1, wherein the interactions between the magnetic material and stone include ionic bonds, van der Waals forces that form surrounding interactions, hydrophobic interactions, adsorption and surface deposition; chemical bonds between the carboxyl-stone, including hydrogen bonds, ester bonds, amide bonds and other covalent bonds; physical and chemical inter-chain entanglement between chains and chemical crosslinking between chains.

A second aspect of the present invention provides a nanoparticle comprising a nanoparticle core comprised of the magnetic material; and a nanoparticle shell formed by linking a surface modifier monomer to the nanoparticle core via an initiator and / or a crosslinking agent.

According to some embodiments of the present invention, the nanoparticle surrounds a stone via physical adsorption, chemical bonding, as well as photosensitive crosslinking, thermosensitive crosslinking and pH-sensitive crosslinking, specifically, the forces for binding and surrounding interactions between the nanoparticle and stone include: van der Waals forces that form surrounding interactions, hydrophobic interactions, adsorption and surface deposition; covalent bonds formed between carboxyl-stone, including hydrogen bonds, ester bonds, amide bonds, and other covalent bonds; physical entanglement and chemical crosslinking between chains.

According to some embodiments of the present invention, the nanoparticle core has a diameter of 2-50 nm, and a weight percentage of 30-95% relative to the whole weight of the nanoparticle, and the magnetic material constituting the core includes a compound of $Fe^{3+}$, $Fe^{2+}$, $Mn^{2+}$ or $Ni^{2+}$, or a metal element selected from iron, nickel, copper, cobalt, platinum, gold, europium, gadolinium, dysprosium, terbium, or a composite or oxide of the aforementioned metals, or any one of the above items or a combination of two or more of the above items, preferably one or a combination of two or more of $Fe^{3+}$, $Fe^{2+}$, $Mn^{2+}$ or $Ni^{2+}$, more preferably $Fe^{3+}$ and $Fe^{2+}$ in a ratio of 15% to 85%, preferably 1: 2.5 to 1.5: 1.It should be noted that the preparation method of the nanoparticle used in the present invention can well control the size of the magnetic nanoparticle core, especially in comparison with the nanoparticles prepared by other methods, among the parameters of the nanoparticles obtained in the present invention, the dispersibility of nanoparticles relative to biomedical applications is very good and less than 1.1.

According to certain embodiments of the present invention, the surface modifier includes a hydrophilic surface modifier with function response, a hydrophobic surface modifier with function response, a photosensitive surface modifier with function response, a thermosensitive surface modifier with function response or a pH sensitive surface modifier with function response, wherein the hydrophilic surface modifier includes acrylic acid, methacrylic acid, isobutyl acrylamide or poly N-substituted isopropylacrylamide; the hydrophobic surface modifier includes olefins, preferably polystyrene, polyethylene or oleic acid; the photosensitive surface modifier is selected from the group consisting of azos and quinolines and benzophenones (PVBP), preferably ethylene benzophenone; the thermosensitive surface modifier is selected from amphiphilic polymers with amide bond, preferably polyacrylamide or poly N-substituted isopropylacrylamide; the pH-sensitive surface modifier is selected from the group consisting of polymers with carboxyl group and quaternary ammonium salt, preferably a polyacrylic acid, dimethylaminoethyl ester and dimethylaminopropyl methacrylate.

According to some embodiments of the present invention, the crosslinking agent includes 3-(methacryloyloxy)propyltriethoxysilane, divinylbenzene, diisocyanate or N,N-methylenebisamide, and the initiator includes 3-chloropropionic acid, CuCl, 4,4'-dinonyl-2,2-bipyridine or potassium persulfate.

The third aspect of the invention provides a method of preparing the nanoparticle, comprising the steps of: a) preparing the nanoparticle core using the magnetic material; and b) forming the nanoparticle shell by in situ linking the surface modifier monomer to the nanoparticle core by the initiator and / or crosslinking agent to form a nanoparticle shell. As used herein, "in situ" means that the surface modifier is directly attached to the surface of the nanoparticle core. The resulting modified nanoparticle has a size between 50 nm and 5000 nm, which varies according to different conditions.

According to some embodiments of the present invention, the nanoparticle core is composed of $Fe_3O_4$, $MnFe_2O_4$, $\gamma$-$Fe_2O_3$, or other nanoscale-sized ferrite particles, and these ferrite particles are prepared by the following steps:

dissolving a proportion of the metal salt-containing material in water;
feeding nitrogen to expel oxygen in the solution;
adding a catalyst at a room temperature of 20-30°C to adjust the pH to 8-12, preferably 10;
keeping agitation and reaction for 20-40 minutes; and
reacting under condition of 50-100°C, preferably 70°C water bath, for 20-40 minutes, then separating with a magnet and drying to obtain the magnetic nanoparticle core.

In a particular embodiment of the present invention, the oxygen-containing metal salt is $FeCl_3 \cdot 6H_2O$ and $FeCl_2 \cdot 4H_2O$, which are dissolved in water in a molar ratio of 15% to 85%, preferably 1: 2.5 to 1.5: 1, wherein the catalyst is ammonia. $Fe_3O_4$ nanoparticles can be obtained by following the above steps.

According to some embodiments of the present invention, the step b) includes dispersing the prepared nanoparticles in an aqueous solution, adding with an xylene solution of 3-chloropropionic acid, polystyrene, CuCl and 4,4'-dinonyl-2,2-bipyridine, wherein the molar ratio between the solution of iron particles and the reaction solution is 1: 1; reacting the mixture for 15-30 hours, preferably 24 hours, at 130 °C under continuous agitation; and collecting the nanoparticles with a magnet, washing with repeatedly with toluene to obtain hydrophobic polystyrene-coated magnetic iron oxide nanoparticles.

According to some embodiments of the present invention, the step b) includes dissolving and dispersing the resulting nanoparticle core into xylene, adding with a silane coupling agent, wherein the silane coupling agent, wherein the nanoparticles, xylene and silane coupling agent are added in a ratio of 95: 5; reacting under the protection of nitrogen atmosphere at 20-100 °C, preferably at 80 °C, for 2-5 hours, preferably for 3 hours; washing with an alcoholic solvent (preferably absolute ethanol) and drying for 12 hours, dispersing in an aqueous solution under ultrasonic condition, adding with potassium persulfate; reacting under nitrogen protection, 40-80 °C for 10 minutes, adding with acrylic acid to continue reaction at 40-80 °C for 1 hour, wherein the reaction temperature is preferably 70 °C; and separating with a magnet, washing and drying to obtain the polyacrylic acid-modified hydrophilic nanoparticles.

According to some embodiments of the present invention, the above step b includes: dissolving and dispersing $Fe_3O_4$ nanoparticles into xylene, and adding with a silane coupling agent, wherein the silane coupling agent (the ratio of the added $Fe_3O_4$ nanoparticles and the silane coupling agent is 95: 5); reacting under protection of nitrogen atmosphere at 80 °C for 2-5 hours, preferably for 3 hours; washing with an alcoholic solvent (preferably absolute ethanol) and drying for 12 hours, dispersing in an aqueous solution under ultrasonic condition, adding with potassium persulfate; reacting under nitrogen protection at 40-80 °C for 10 minutes, adding with a photosensitive monomer such as vinyl benzophenone, a thermosensitive monomer such as N-isopropylacrylamide, or a pH-sensitive monomer such as dimethylaminopropyl methacrylate, etc. (or a blended monomer of acrylic acid and styrene), reacting continuously for 1 hour at 40-80 °C, preferably at 70 °C; and separating with a magnet, washing and drying to obtain the photosensitive, thermosensitive or pH sensitive functional monomer-modified magnetic nanoparticles, respectively.

In certain embodiments of the present invention, the photosensitive monomer modification based on the hydrophilic surface modification includes: dissolving and dispersing the polyacrylic acid-modified magnetic nanoparticles in an alcoholic solvent, dispersing for 5-30 minutes under ultrasonic condition, then adding an initiator and a photosensitive monomer polyvinylbenzophenone, refluxing and reacting at agitation and 130 °C for 24 hours under condition that oxygen is kept out to prepare the photosensitive monomer-modified magnetic nanoparticles.

In the above embodiment, when aqueous ammonia is used as catalyst to prepare the nanoparticles, the method for dropping aqueous ammonia is performed in a continuous and dropwise manner with assistance of an electronic pump at a speed of 20-100 drops / minute, preferably 40-60 drops / minute; and when the magnetic material is an element material, the liquid monomer is added in a dropwise and continuous manner with assistance of an electronic pump, and the reaction is carried out under agitation with a speed of 100-1000 revolutions / minute, preferably 500-700 revolutions / minute.

It should be noted here that the particle size, distribution and morphology (such as shape of sphere, rod, diamond, etc.) of the obtained magnetic nanoparticle core can be relatively easily controlled under the synthetic methods and preparation conditions as we designed above. Furthermore, the surface-modified magnetic nanoparticles prepared by the above method have particle size and distribution superior to those of magnetic nanoparticles obtained by conventional preparation methods. As shown in the following table, the dispersibility index (PD.I.) of the obtained nanoparticles is basically close to 1.0, which clearly shows that the particle size distribution of the obtained particles is narrow. This is very important because, for in vivo biomedical applications, the size and dispersion of nanoparticles determine the breadth of their medical applications. The PD.I described herein for describing the dispersibility of nanoparticles is defined as follows:

$$PD.I. = <Rh^2> / <Rh>^2$$

wherein, Rh represents hydrodynamic radius of particle.

The nanoparticle core and the surface-modified magnetic nanoparticle have distribution PD.I. as shown in the following table:

|  | Magnetic nanoparticle core | After surface modification |
|---|---|---|
| Diameter/nm | 40-50 | 80-100 |
| PD.I./a.u. | 0.005 | 0.0055 |

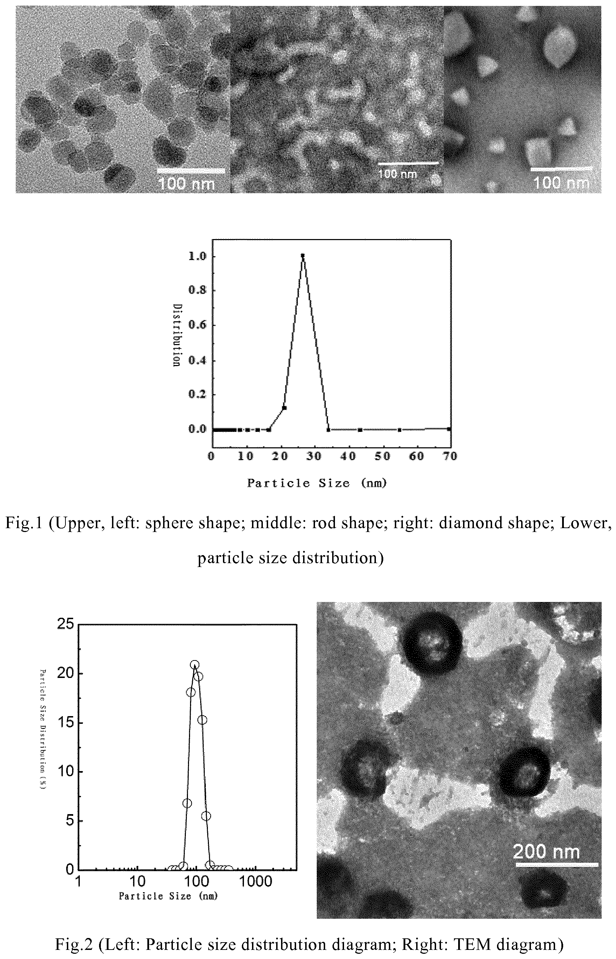

In addition, as shown in Fig.2, the structure of the nanoparticles obtained in the present invention is clear.

According to a fourth aspect of the present invention, there is provided a device for removal of stone, which can be used in the urinary system and consists of a magnetic nanoparticle in combination with a stone-removal magnetic probe rod system. The device for removal of stone comprises the above-mentioned nanoparticles of the present invention and an auxiliary stone-removal magnetic probe rod system. The device for removal of stone can be used for removing a stone in urinary system such as kidney stone, ureteral stone and bladder stone, etc., as well as removing a stone in human biliary system and a stone-like particle in other organs.

Specifically, the stone-removal magnetic probe rod system comprises a handle, a flexible rod, a magnetic field source, a magnetically permeable material section, etc., in which the handle may be provided with an AC or DC power supply, a power switch, a DC battery compartment and an AC plug; the flexible rod is made of a polymer material including, for example, PU, TPU, PE, PVC, NYLON, PEBAX and silicone rubber and the modified materials of the above materials, the magnetic field source made of a permanent magnet or electromagnet can be embedded in the flexible rod, and optionally a high performance magnetically permeable material is connected to the magnetic field source to form the flexible probe rod with different configurations, for example, the permanent magnet is placed in the middle or back end of the flexible rod, and the magnetically permeable material is placed in the distal end of the flexible rod, so that such configuration is more conducive to the treatment of renal stones under ureteroscope to avoid the situation that the distal end of the flexible rod becomes rigid due to the rigid structure of the permanent magnet or the electromagnet, so that such magnetic probe rod can be successfully inserted into the working channel of ureteroscope, and inserted into upper, middle and lower kidney calices to carry out stone removal operation when driven by the ureteroscope.

In a fifth aspect of the invention, there is provided a use of the nanoparticle of the present invention in manufacture of an article, in which the nanoparticle is prepared in the form of a solution or powder.

The invention provides a novel preparation process for synthesizing hydrophilic, hydrophobic, thermosensitive and pH-sensitive as well as photosensitive magnetic nanoparticles, which has the advantages of simple preparation process, good repeatability and convenient application. By the hydrophobic interaction between the prepared hydrophobic nanoparticles and stones, the chemical bond interaction between hydrophilic nanoparticles and stones, and the polymerization of the photosensitive nanoparticles under illumination, the stones are surrounded; and the thermosensitive and pH-sensitive nanoparticles can surround stones through physically surrounding action in ureter; thereby, small stone remaining in body can be removed quickly without damage from the body under the action of an externally applied magnetic field, that is, the stone can be drawn and moved without injuring ureteral wall, and meanwhile the nanoparticles are placed conveniently without shift.

The present invention is further described with reference to the accompanying drawings.

## Brief Description of the Drawings

Fig. 1 shows the transmission electron microscope (TEM) images and the particle size distribution diagrams under dynamic light scattering for the cores with different morphologies obtained in Example 1 of the present invention.

Fig.2 shows the particle size distribution diagram under dynamic light scattering for the monomer-modified nanoparticles obtained in Example 4 of the present invention; and TEM images of the monomer-modified nanoparticles obtained in Example 3 of the present invention.

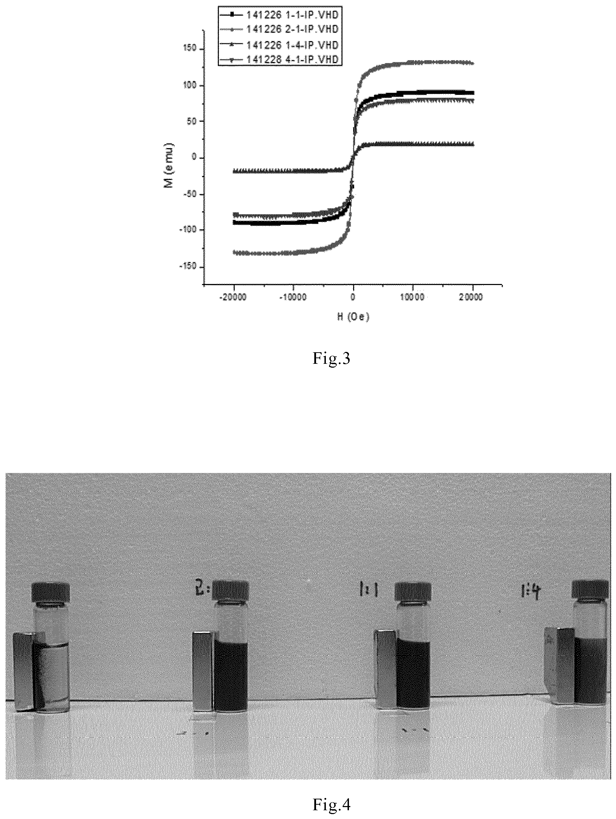

Fig.3 shows the hysteresis curves of the monomer-modified nanoparticle cores with different $Fe^{3+}/Fe^{2+}$ ratios in the synthesis of the nanoparticles obtained in Example 3 of the present invention.

Fig.4 shows the graphs of stone separation for the photosensitive monomer-modified nanoparticle cores obtained in Example 4 of the present invention, and the graphs of separation performance for the nanoparticle cores synthesized with different $Fe^{3+}/Fe^{2+}$ ratios and modified with functional monomer.

Fig.5 shows a graph of biocompatibility to 293t cells for the functional magnetic nanoparticles obtained in Example 4 of the present invention.

Fig.6 shows a graph of stone separation with in vitro assistance for the nanoparticles obtained in Example 6 of the present invention.

Fig.7 shows a graph of safety evaluation in body of animals for the nanoparticles of the present invention.

Fig.8 shows an overall schematic diagram of the magnetic probe rod system for assisting stone removal in the present invention.

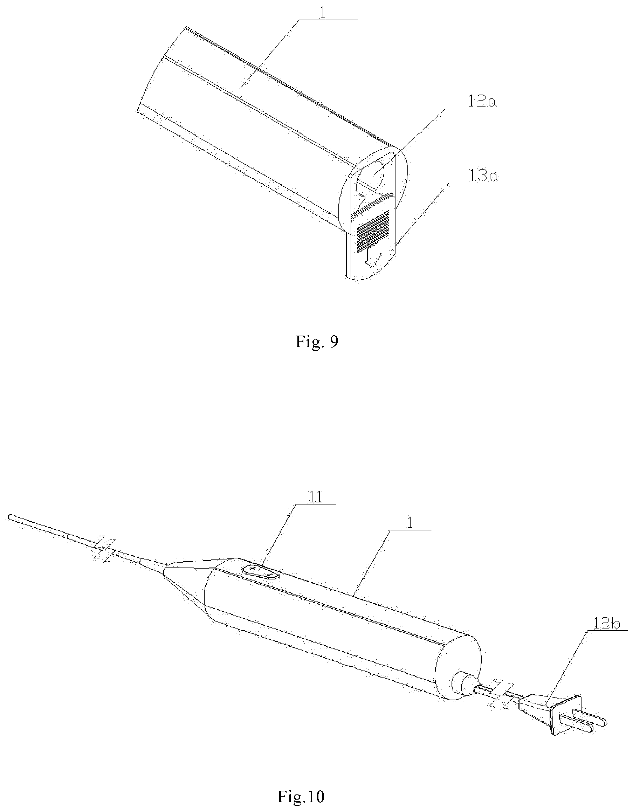

Fig.9 shows a schematic view of the handle portion of the magnetic probe rod system for assisting stone removal in the present invention.

Fig. 10 shows a schematic view of the magnetic probe rod system with an AC power supply for assisting stone removal according to the present invention.

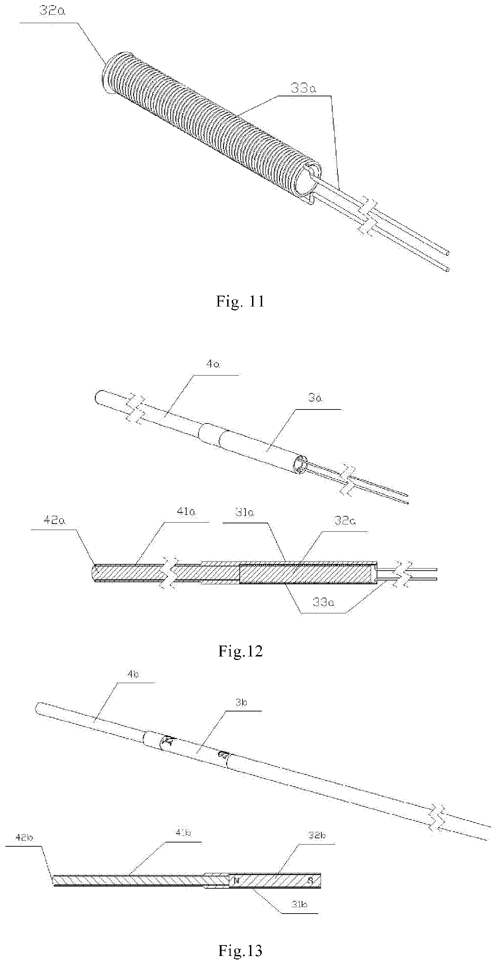

Fig.11 shows a schematic view of the internal structure of the magnetic probe rod system using an electromagnet as magnetic field source according to the present invention.

Fig. 12 shows a schematic view of the magnetic probe rod system of the present invention which uses an electromagnet as magnetic field source and a magnetically permeable material section at distal end.

Fig.13 shows a schematic view of the magnetic probe rod system of the present invention which uses a permanent magnet as magnetic field source and a magnetically permeable material section at distal end.

Fig. 14 shows a schematic view of the magnetic probe rod system of the present invention which uses a permanent magnet as magnetic field source and has not a magnetically permeable material section at distal end.

Fig. 15 shows a diagram of the principle of interaction between magnetic particles and stones.

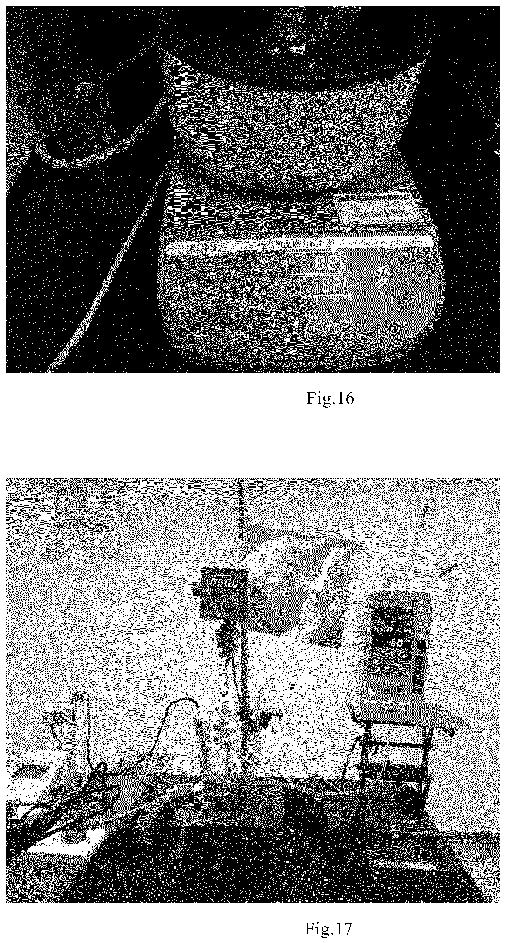

Fig.16 shows a diagram of agitation of the reaction system in steps 2-4) of the method for preparing the nanoparticles of the present invention, which uses a magnetic stirrer at a speed of 100-1000 revolutions / minute, preferably 500-700 revolutions / minute.

Fig.17 shows a diagram of the steps 1-4) of the method for preparing the nanoparticles according to the present invention, in which aqueous ammonia and liquid monomer are added dropwise continuously and uniformly by an electronic pump at a rate of 20-100 drops / minute, preferably 40-60 drops / minute.

Fig.18 shows a diagram of comparison of stone removal performance for the different methods.

Fig.19 shows a diagram of in vivo stone removal performance for two mimic situations.

**Specific Models for Carrying Out the Invention**

The present invention is further illustrated below with reference to the accompanying drawings and specific examples. It should be understood that the following examples are only used to illustrate the present invention, rather than to limit the scope of the present invention.

According to a first aspect of the present invention, there is provided a use of a magnetic material for removal of stones, and further a use of a magnetic nano-material for removal of stones. Meanwhile, the present invention provides a magnetic nano-material capable of safely and efficiently removing stones in urinary system, in which the magnetic nano-material is a functional magnetic nanoparticle comprising, for example, hydrophilic, hydrophobic, photosensitive, thermosensitive, pH-sensitive magnetic nanoparticles, which morphology can be spherical, rod-shaped and the like, which structure can be of core-shell structure consisting of a magnetic core and a monomer modifier such as hydrophilic, hydrophobic, temperature sensitive, pH-sensitive or photosensitive surface modifiers as well as a small amount of initiator; in which the hydrophilic surface modifier forms a hydrophilic shell by polymerization to surround the magnetic nanoparticle core, including the hydrophilic materials with positive charges, negative charges and electricity neutrality; the hydrophobic surface modification is carried out by a poor water-soluble polymer or inorganic material; the other functional materials such as photosensitive, thermosensitive and pH-sensitive monomer modifiers may be polymerized by a crosslinking agent and embedded in a hydrophobic shell, or these monomer modifiers may be in situ and directly attached to the surface of the core by an initiator and / or crosslinking agent.

Among the various kinds of responsive magnetic nanoparticles such as hydrophilic, hydrophobic, thermosensitive, pH-sensitive and photosensitive magnetic nanoparticles, the materials for synthesizing magnetic nanoparticle cores comprise $Fe^{3+}$, $Fe^{2+}$ and $Mn^{2+}$, $Ni^{2+}$ compounds, as well as metal elements such as iron (Fe), nickel (Ni), copper (Cu), cobalt (Co), platinum (Pt), gold (Au), europium (Eu), gadolinium (Gd), dysprosium (Dy), terbium (Tb), or composites and oxides of the metals, such as $Fe_3O_4$ or $MnFe_2O_4$, preferably iron, manganese or their compounds; also preferably, any one of them or any combination of two or more of them may be used; and the core has a size of 2-50 nm.

The preparation method of the magnetic nanoparticle core includes co-precipitation methods, emulsion methods, redox reaction or high-temperature high-pressure methods. The weight percentage of the magnetic nanoparticle core accounts for 30% to 95% relative to the total weight of the functional magnetic nanoparticle, taking the synthesis of $Fe_3O_4$ as example, the ratio of $Fe^{3+}$ to $Fe^{2+}$ is 15% to 85%, preferably 1: 2.5 to 1.5: 1 for $Fe^{3+}$ and $Fe^{2+}$.

The surface of the magnetic nanoparticle can be subjected to functional modification, such as hydrophilic modification, hydrophobic modification, and modification with photosensitive, thermosensitive and pH-sensitive materials.

According to a first embodiment of the present invention, there is provided a hydrophilically modified functional particle, wherein the core size is 2-50 nm, the magnetic nano-core has a weight of 30-95% relative to the total nanoparticle; the hydrophilic surface modifier is a polycationic or an anionic polymer, such as acrylic acid, methacrylic acid and isobutyl acrylamide, etc., which weight percentage is 2-8% relative to the whole hydrophobic magnetic nanoparticle. The magnetic core particle is attached on its surface with an initiator such as 3-chloropropionic acid or the like, then a polymer based on acrylic acid, methacrylic acid and isobutyl acrylamide or the like is modified on the particle surface with a crosslinking agent by a chemical reaction, such as radical, ring-opening polymerization and atom transfer radical polymerization (ATRP); the shape of the particle may be spherical, rod-shaped and layered, preferably spherical particles. The crosslinking agent is 3-(methacryloyloxy) propyltriethoxysilane (MPS), divinylbenzene and diisocyanate or N,N-methylenebisacrylamide (MBA) and the like.

According to a second embodiment of the present invention, there is provided a hydrophobically modified functional particle, wherein the core has a size of 2-50 nm, the magnetic nano-core is 30-95% by weight relative to the whole nanoparticle; the hydrophobic surface modifier is an water insoluble monomer, such as olefins, for example, polystyrene and the like, which weight percentage is 2-8% by weight relative to the whole hydrophobic magnetic nanoparticle. The magnetic core particle is attached to its surface with an initiator such as 3-chloropropionic acid, and then a hydrophobic polymer based on styrenes is modified on the particle surface by a crosslinking agent via a chemical reaction such as radical, ring-opening polymerization and atom transfer radical polymerization (ATRP); the morphology of the particle may be spherical, rod-shaped and layered, preferably spherical particle. The crosslinking agent is 3-(methacryloyloxy) propyl triethoxysilane (MPS), divinylbenzene and diisocyanate or N,N-methylenebisacrylamide (MBA) and the like.

According to a third embodiment of the present invention, there is provided a photosensitive surface-modified functional particle, wherein the core has a size of 2-50 nm, and the magnetic core is 30-95% by weight relative to the whole nanoparticle; the photosensitive modifier is selected from the group consisting of azos and quinolines as well as benzophenones (PVBP), etc., which weight percentage is 2-8% relative to the whole hydrophobic magnetic nanoparticle. The magnetic core particle is attached to its surface with an initiator such as 3-chloropropionic acid, and then a photosensitive polymer based on benzophenone (PVBP) and the like is modified to the surface of the particle by a crosslinking agent via a chemical reaction such as radical, ring-opening polymerization and atom transfer radical polymerization (ATRP); the morphology of the particle may be spherical, rod-shaped and layered, preferably spherical particle. The crosslinking agent is 3-(methacryloyloxy) propyltriethoxysilane (MPS), divinylbenzene and diisocyanate or N,N-methylenebisacrylamide (MBA) and the like.

According to a fourth embodiment of the present invention, there is provided a thermosensitive surface-modified functional particle, wherein the size of the core is 2-50 nm, and the magnetic nano-core is 30-95% by weight relative to the whole nanoparticle; the thermosensitive surface modifier is selected from the group consisting of amphiphilic polymers carrying amide bonds, such as polyacrylamide, poly N-substituted isopropylacrylamide, etc., which weight percentage is 2-8% relative to the whole hydrophobic magnetic nanoparticle. The magnetic core particle is attached to its surface with an initiator such as 3-chloropropionic acid, and then a thermosensitive polymer such as poly N-substituted isopropylacrylamide is modified to the surface of the particle by a crosslinking agent via a chemical reaction such as radical, ring-opening assembly and atom transfer radical polymerization (ATRP); the shape of the particle may be spherical, rod-shaped and layered, preferably spherical particle. The crosslinking agent is 3-(methacryloyloxy) propyltriethoxysilane (MPS), divinylbenzene and diisocyanate or N,N-methylenebisacrylamide (MBA) and the like.

According to a fifth embodiment of the present invention, there is provided a pH-sensitive surface-modified functional particle, wherein the core has a diameter of 2 to 50 nm, the magnetic nano-core is 30 to 95% by weight relative to the whole nanoparticle; the pH-sensitive surface modifier is selected from the group consisting of polymers carrying carboxyl groups and quaternary ammonium salt groups, such as polyacrylic acid, dimethylaminoethyl ester and dimethylaminopropyl methacrylate, etc., which weight percentage is 2-8% relative to the whole hydrophobic magnetic nanoparticle. The magnetic core particle is attached to its surface with an initiator such as 3 -chloropropionic acid, and then a pH-sensitive polymer based on dimethylaminoethyl methacrylate and dimethylaminopropyl methacrylate or the like is modified to the surface of the particle by a crosslinking agent via a chemical reaction such as radical, ring-opening polymerization and atom transfer radical polymerization (ATRP); the shape of the particle may be spherical, rod-shaped and layered, preferably spherical particle. The crosslinking agent is 3-(methacryloyloxy) propyltriethoxysilane (MPS), divinylbenzene and diisocyanate or N,N-methylenebisacrylamide (MBA) and the like.

In the above embodiments of the present invention, an initiator and / or a crosslinking agent are further included. The initiator includes thermal initiators, for example, potassium persulfate, ammonium persulfate and azo type initiators; the crosslinking agent includes 3-(methacryloyloxy) propyltriethoxysilane (MPS), divinylbenzene and diisocyanate or N,N-methylenebisacrylamide (MBA), molecular weight is 100,000, and oleic acid, etc.

According to a second aspect of the present invention, there is provided a preparation method for a nanoparticle obtained from a magnetic material. The preparation method generally includes two main steps: synthesis of a magnetic nanoparticle core (the magnetic material constitutes the nanoparticle core), and various surface modifications based on the magnetic nanoparticle core (hydrophilic, hydrophobic and thermosensitive, photosensitive and pH-sensitive modification). Taking the preparation of magnetic $Fe_3O_4$ nanoparticles as an example, the two steps of the preparation method are respectively described in detail.

1) Preparation of magnetic $Fe_3O_4$ nanoparticle core

$FeCl_3 \cdot 6H_2O$ and $FeCl_2 \cdot 4H_2O$ in a certain molar ratio (the molar ratio of $FeCl_3 \cdot 6H_2O$ and $FeCl_2 \cdot 4H_2O$ is 15% to 85%, preferably 1: 2.5 to 1.5: 1) are dissolved in 100 mL of water, fed with nitrogen gas to expel oxygen in the solution, added with aqueous ammonia at a room temperature of 20-30°C to adjust the pH value of 8-12, preferably 10, and kept agitation and reaction for 20-40 minutes; then under a 50-100 °C, preferably 70 °C water bath, the reaction is carried out for 20-40 minutes, and then $Fe_3O_4$ nanoparticles are obtained by separation with a magnet and drying. There are different kinds of preparation methods such as co-precipitation method, thermal decomposition method, hydrothermal synthesis method, microemulsion method (reverse micelle method) and the like.

2) Surface modification of the synthesized $Fe_3O_4$ nanoparticle core

2.1) Hydrophobic modification of the surface of the synthesized $Fe_3O_4$ nanoparticle core

The $Fe_3O_4$ nanoparticle core prepared in the step 1) is dispersed into an aqueous solution, added with an initiator 3-chloropropionic acid and pre-treated for 12 hours, then added with an xylene solution of a hydrophobic surface-modifying monomer polystyrene and an active initiator CuCl and 4,4'-dinonyl-2,2-dipyridine (the molar ratio of the iron particle solution and the reaction solution is 1:1), and the mixture solution is reacted under continuous agitation at 130 °C for 15-30 hours, preferably 24 hours; the resulting nanoparticles are collected with a magnet and washed repeatedly with toluene to obtain the hydrophobic polystyrene-coated magnetic iron oxide nanoparticles.

Here, 3-chloropropionic acid is used as an initiator, and CuCl and 4,4'-dinonyl-2,2-bipyridine are used as another initiator. In addition, according to one embodiment of the present invention, the reaction time is preferably 18 to 30 hours, preferably 24 hours. In addition, according to one embodiment of the present invention, the solvent is toluene or xylene in an amount of 1/2 to 1 of the monomer volume, the mass ratio of the surface-modified polystyrene magnetic nanoparticles, the initiator and the monomer is 95: 0.5: 4.5.

2.2) Hydrophilic modification of the surface of the synthesized $Fe_3O_4$ nanoparticle core

The $Fe_3O_4$ nanoparticle core obtained in the above step 1) is dissolved and dispersed into xylene, added with a silane coupling agent (the addition ratio of $Fe_3O_4$ nanoparticles and the silane coupling agent is 95: 5), and reacted at 80 °C under nitrogen protection for 2-5 hours, preferably 3 hours; then washed with an alcoholic solvent (preferably absolute ethanol) and dried for 12h, dispersed in an aqueous solution under ultrasonic condition, added with potassium persulfate; reacted under nitrogen protection at 40-80 °C for 10 minutes, then added with acrylic acid and reacted continuously at 40-80 °C for 1 hour, preferably reacted at a reaction temperature of 70 °C; separated by a magnet, washed and dried to obtain polyacrylic acid-modified, hydrophilic surface-modified magnetic nanoparticles.

Here, the silane coupling agent is 3-(methacryloyloxy)propyl triethoxysilane (MPS) in an amount of 8 to 16 times the mass of acrylic acid; the solvent is benzene or 2-toluene; potassium persulfate is used as initiator; the reaction time is preferably 20 minutes to 80 minutes. According to one embodiment of the present invention, the mass ratio of the surface-modified magnetic $Fe_3O_4$ nanoparticles, the potassium persulfate and the acrylic acid monomer is 25-100: 1: 100.

In addition, the alcoholic solvent here is methanol, ethanol or butanol, preferably ethanol, the reaction temperature is preferably 100 °C to 150 °C, the reaction time is preferably 18 hours to 24 hours, and the mass ratio of the photosensitive monomer-modified magnetic nanoparticles, the potassium sulfate and the vinylbenzophenone monomer is 25-100: 1: 100.

2.3) Functional modification of the surface of the synthesized $Fe_3O_4$ nanoparticle core

The $Fe_3O_4$ nanoparticle core as prepared in the above step 1) is dissolved and dispersed into xylene, added with a silane coupling agent (the addition ratio of the $Fe_3O_4$ nanoparticles and the silane coupling agent is 95: 5), reacted under nitrogen protection at 80 °C for 2-5 hours, preferably 3 hours, then washed with an alcoholic solvent (preferably absolute ethanol) and dried for 12 hours, dispersed into an aqueous solution under ultrasonic condition, added with potassium persulfate; reacted under nitrogen protection at 40-80 °C for 10 minutes, then added with a photosensitive monomer vinyl benzophenone, or a thermosensitive monomer N-substituted isopropylacrylamide, or a pH-sensitive monomer dimethylaminoethyl methacrylate, reacted at 40-80 °C for 1 hour, preferably reacted at a reaction temperature of 70 °C; separated by a magnet, washed and dried to obtain magnetic nanoparticles with photosensitive, thermosensitive or pH-sensitive surface modification, respectively.

In addition, the functionally modified nanoparticles here may also be obtained by a cross-reaction with steps 2.1 and 2.2) and step 3) after pre-modification of the surface of the nanoparticle in the steps 2.1 and 2.2). That is, after the modification of 3-chloropropionic acid in step 2.1 (or the modification of silane coupling agent in step 2.2), the hydrophobic monomer styrene (or hydrophilic monomer acrylic acid) and the functional monomer such as vinyl benzophenone, N-substituted isopropylacrylamide or dimethylaminoethyl methacrylate and the like are added at the same time, and reacted at 40-80 °C for 1 hour, preferably reacted at a reaction temperature of 70 °C; separated by a magnet, washed and dried to obtain magnetic nanoparticles with photosensitive, thermosensitive or pH-sensitive surface modification. The co-reaction would result in co-modified functional nanoparticles corresponding to polystyrene (or polyacrylic acid) and the functional monomer.

Here, the silane coupling agent is 3-(methacryloyloxy)propyl triethoxysilane (MPS) in an amount of 8 to 16 times the mass of acrylic acid; the solvent is benzene or 2-toluele in an amount of 8 to 16 times the mass of acrylic acid; potassium persulfate is used as initiator; the reaction time is preferably 20 minutes to 80 minutes. According to one embodiment of the present invention, the mass ratio of the surface-modified magnetic $Fe_3O_4$ nanoparticles, the potassium persulfate and the acrylic acid monomer is 25-100: 1: 100.

In addition, the alcoholic solvent here is methanol, ethanol or butanol, preferably ethanol, the reaction temperature is preferably 100 °C to 150 °C, and the reaction time is preferably 18 hours to 24 hours. The mass ratio of the functional monomer-modified magnetic nanoparticles, the potassium persulfate and the functional monomer is 25-100: 1: 100.

In the process for preparing the nano iron oxide (in step 1), the $Fe_3O_4$ nanoparticles are nanoscale ferroferric oxide particles ($Fe_3O_4$), $MnFe_2O_4$, nanoscale ferric oxide particles ($\gamma$-$Fe_2O_3$) or other nanoscale ferrite particles , the aqueous ammonia is used as catalyst, the reaction pH is preferably 9 to 10, the reaction time is preferably 20 to 30 minutes, the reaction temperature is between 50-100 °C, preferably 70-80 °C, the preferred ratio of $Fe^{3+}$:$Fe^{2+}$ is 15% to 85%, preferably 1.5: 1 to 1: 2.5.

In addition, in the above process for preparing nanoscale iron oxide (i.e., step 1)), the nanoparticle core is nanoscale ferroferric oxide particles ($Fe_3O_4$). Those skilled in the art can understand that it is also possible to use $MnFe_2O_4$, nanoscale ferric oxide particles ($\gamma$-$Fe_2O_3$) or other nanoscale ferrite particles. The aqueous ammonia is used as catalyst, the reaction pH is preferably 9 to 10, the reaction time is preferably 20-30 minutes, the reaction temperature is between 50-100 °C, preferably 70-80 °C. The preferred ratio of $Fe^{3+}:Fe^{2+}$ is 15% to 85%, preferably 1: 2.5 to 1.5: 1.

In addition, in the above embodiments of the present invention, the agitation in the above reaction system is performed by a magnetic stirrer at a speed of 100-1000 revolutions / minute, preferably 500-700 revolutions / minute.

In addition, in the above embodiments of the present invention, the aqueous ammonia and the liquid monomer are added dropwise continuously and uniformly by an electronic pump at a rate of 20-100 drops / minute, preferably 40-60 drops / minute. Through the use of electronic pump for continuous and uniform dripping, large-scale production can be easily achieved, and the dispersibility and uniformity of nanoparticles can be well controlled.

According to a third aspect of the present invention, there is provided a magnetic probe rod system for assisting the removal of stone in urinary system. The magnetic probe rod system comprises a handle 1, a flexible rod 2, a magnetic field source 3 and a magnetically permeable material section 4 (in the present invention, the side of the handle is defined as the proximal end of the instrument, and the end of the magnetic field source is defined as the distal end). When an electromagnet is selected as the magnetic field source 3, a switch 11 can be integrated into the handle 1, and the magnetic field power supply can be selected from DC battery, and a battery compartment 12a and a battery cover 13a are provided accordingly; when an AC power is selected as the magnetic field power supply, an AC power plug 12b is provided on the handle correspondingly. When the magnetic field source is an electromagnet, the magnetic field source 3 is composed of an electromagnet core 32a and an electromagnetic coil 33a, and externally provided with a magnetic field source encapsulation membrane 31a made by biocompatible material; when the magnetic field source 3 is a permanent magnet, the magnetic field source 3 is composed of the permanent magnet 32b and the magnetic field source encapsulation membrane 31b on its surface; in order to ensure the accessibility of the present invention in human body, a magnetically permeable material section 4 can be optionally disposed at the distal end of the magnetic field source 3, that is, when the electromagnet is used as the magnetic field source, the magnetically permeable material section 4a can be optionally disposed at the distal end of the electromagnet 3a, and the magnetically permeable material section is composed of a highly magnetically permeable material 42a and a magnetically permeable material encapsulating membrane 41a, in which the highly magnetically permeable material 42a can be made of an iron-based magnetically permeable material, preferably a pure iron material, and the magnetically permeable material encapsulating membrane 41a and the magnetic field source encapsulation membrane 31a can be made of the same material; when the permanent magnet is selected as the magnetic field source, the magnetically permeable material section 4b may also be disposed at the distal end of the permanent magnet 3b, composed of the highly permeable material 42b and the magnetically permeable material encapsulation membrane 41b, and the magnetically permeable material encapsulation membrane 41b and the magnetic field source encapsulation membrane 31b can be made of the same material; when it is not necessary to dispose a magnetically permeable material section at the distal end of the magnetic field source, the system of the present invention is composed of the handle 1, the flexible rod 2 and the magnetic field source 3; for example, when the permanent magnet is selected as the magnetic field source, the magnetic field source at distal end would be composed of a permanent magnet 32c and an externally disposed magnetic field source encapsulation membrane 31c; in the above embodiment, the flexible rod 2 may be made of a polymer material such as PU, TPU, PE, PVC, NYLON, PEBAX and silicone rubber, as well as modified materials of the above materials, and the magnetic field source encapsulation membranes 31a, 31b and 31c as well as the magnetically permeable material encapsulation membranes 41a and 41b are all made of the same material as the flexible rods.

Further, the present invention provides a use of a magnetic nanoparticle in manufacture of an article, in which the magnetic nanoparticle is further processed to form a stone-removing solution (using physiological saline, buffer as solvent) or a stone-removing powder, preferably a stone-removing solution, which is used as a medical clinical article.

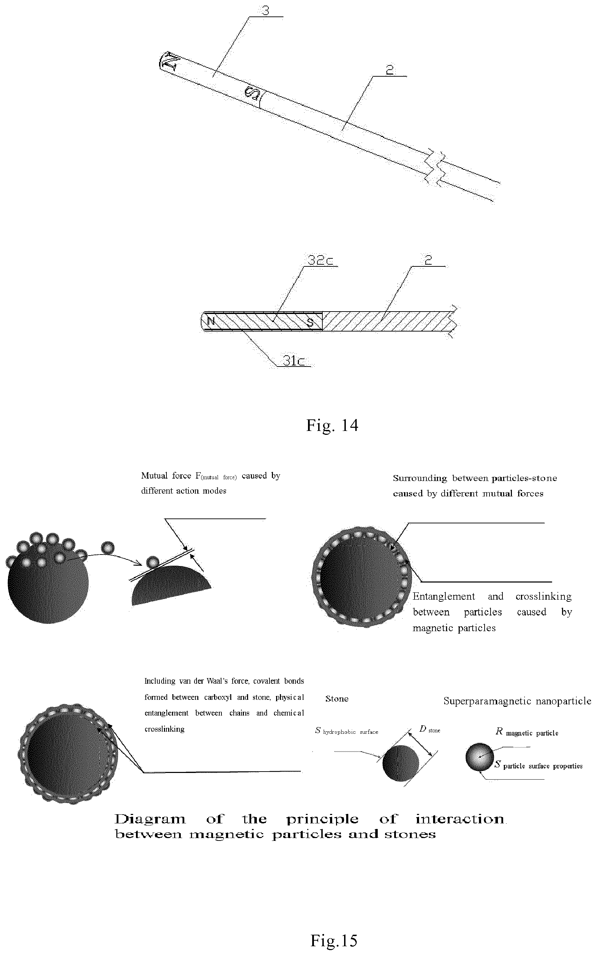

The basic principle of the high performance system for removing a stone in urinary system, which consists of the functional magnetic nanoparticle composed of the magnetic material and the magnetic probe rod as shown, is achieved by the following steps: 1) pulverization of stones in vivo; 2) injection of the functional magnetic nanoparticles, the magnetic nanoparticles having excellent dispersibility (dispersibility and dispersion coefficient are related, the smaller the dispersion coefficient, the better the dispersibility); 3) interaction between the functional magnetic nanoparticles and the stones; 4) surrounding the stones with the functional magnetic nanoparticles; 5) physical or chemical crosslinking of the magnetic nanoparticles on the surface of the stones; 6) removal of the magnetized stones under the guidance of an external magnetic field. Among them, the stones are surrounded and magnetized by the magnetic nanoparticles via physical adsorption, chemical bonding and the like. The physical adsorption mainly refers to an attraction generated by van der Waal's force and hydrophobic interaction within the range of action between hydrophobic magnetic particles - particles and between particles - stones, so that the surface of the stones are adsorbed and surrounded by the magnetic particles; the chemical bonding mainly refers to the interaction between the hydrophilic magnetic nanoparticles - particles and between particles - stones mainly through the formation of chemical bonds (chemical bonds, such as hydrogen bonds, covalent bonds, etc., between carboxyl on the surface of the particles and the stones), so that the surface of the stones is surrounded with the magnetic particles; the chemical bonding includes: the functional magnetic nanoparticles (such as photosensitive, thermosensitive nanoparticles, etc.) firstly act on the stones via physical adsorption, and then further enhance the mutual forces between particles - particles and particles - stones via photosensitive crosslinking, thermosensitive physical entanglement (crosslinking) and the like to surround the stones. The principle of the above interaction is shown in Fig.15:

The followings are detailed examples of the structure, preparation and use of the magnetic nanoparticles of the present invention.

Example 1: Preparation of magnetic nanoparticle core

1. Preparation of 10 nm $Fe_3O_4$ by co-precipitation method

3.05g $FeCl_3.6H_2O$ and 2.08g $FeCl_2.4H_2O$ (molar ratio 1: 1) were dissolved in 50ml deionized water in a three-necked flask. Nitrogen gas was used throughout the experiment. Aqueous ammonia was added dropwise with a syringe and stirred vigorously at room temperature to adjust pH to 9, the solution gradually turned from yellow to brown and finally black, and the reaction was carried out for 20 minutes. After the reaction, the solution was placed in a 70 °C water bath and incubated for 20 minutes, stirred vigorously to remove excess ammonia. The three-necked flask was taken out and allowed to cool to room temperature under vigorous agitation. The suspension of the synthesized $Fe_3O_4$ magnetic particles was poured into a 50ml centrifuge tube, a powerful magnet was used to attract the magnetic particles, liquid waste was discarded, deionized water was added, the magnetic particles were re-suspended under ultrasonic, so repeatedly to wash away excess ammonia until pH showed neutral. The collected magnetic particles were placed in an oven at 65 °C to be dried and dewatered. The synthesized magnetic particles were weighed, and 1.0ml suspension of 0.02mg / ml magnetic particles was formulated for measurement of particle size. A total of 1.5ml of 0.02M sodium oleate solution was added dropwise to 1.0ml suspension of 0.2mg / ml magnetic particles, reacted at 70 °C under nitrogen and vigorous agitation for 30 minutes, and then cooled to room temperature. Excess sodium oleate was removed by dialysis using 12KD dialysis membrane. Thus, 1.0ml of 0.02mg / ml sodium oleate-encapsulated $Fe_3O_4$ solution was formulated, and its particle size was measured.

2. Preparation of $Fe_3O_4$ magnetic nanoparticle core

2.1 Thermal decomposition method

2.1.1 Synthesis of 4nm $Fe_3O_4$ seeds:

5 mmol of ferric triacetylacetonate, 5 mmol of 1,2-dihydroxyhexadecane, 3 mmol of oleic acid, 1 mmol of oleylamine were dissolved in 20 ml of diphenyl ether and magnetically stirred under a nitrogen atmosphere. The above mixture was stirred at 200 °C for 30 minutes, and then heated under reflux for 30 minutes at 265 °C under the protection of nitrogen gas. The heating was stopped, and the dark brown liquid mixture obtained from the reaction was cooled to room temperature; under atmospheric conditions, 400 ml of ethanol was added, and the resulting black material was separated by overspeed centrifugation. The black product obtained by centrifugation was redissolved in n-hexane containing 50 $\mu$L of oleic acid and 50 $\mu$L of oleylamine, centrifuged at 600 rpm for 10 minutes to remove insoluble residues. The resulting 4 nm $Fe_3O_4$ product was precipitated with ethanol, centrifuged at 600 rpm for 10 minutes to remove solvent and then redispersed into n-hexane. The following different methods were used respectively to synthesize surface-functionalized nanoparticles with different sizes.

2.1.2 Synthesis of 6 nm $Fe_3O_4$ nanoparticle core using 4 nm $Fe_3O_4$ seeds

20 mmol of ferric triacetylacetonate, 10 mmol of 1,2-dihydroxyhexadecane, 6 mmol of oleic acid and 6 mmol of oleylamine were dissolved in 20 ml of diphenyl ether and magnetically stirred under a nitrogen atmosphere. The above mixture was heated at 200 °C for 2 hours, and then heated under reflux for 1h at 300 °C under the protection of nitrogen gas. The heating was stopped, and the dark brown liquid mixture obtained from the reaction was cooled to room temperature. The aforementioned operation steps for the synthesis of the 4 nm $Fe_3O_4$ particles were adopted to obtain a black brown suspension of 6nm $Fe_3O_4$ particles dispersed in n-hexane.

2.1.3 Synthesis of 8nm $Fe_3O_4$ nanoparticle core using 6nm $Fe_3O_4$ seeds

2 mmol of ferric triacetylacetonate, 10 mmol of 1,2-dihydroxyhexadecane, 2 mmol of oleic acid and 2 mmol of oleylamine were dissolved in 20 ml of ethyl ether and magnetically stirred under nitrogen protection. 84 mg of 6 nm $Fe_3O_4$ particles were weighed, dissolved in 4 ml of n-hexane, and then added to the above mixture liquid. The above mixture liquid was first heated at 100 °C for 30 minutes to remove n-hexane, then heated at 200 °C for 1 hour, and heated under reflux at 300 °C for 30 minutes under nitrogen protection. The heating was stopped, and the black mixture liquid resulting from the reaction was allowed to cool to room temperature. The aforementioned synthesis steps for 4 nm $Fe_3O_4$ particles was used to give a dark brown suspension of 8 nm $Fe_3O_4$ particles dispersed in n-hexane. Similarly, 80 mg of 8 nm $Fe_3O_4$ seeds reacted with 2 mmol of ferric triacetylacetonate and 10 mmol of 1,2-dihydroxyhexadecane to produce 10 nm $Fe_3O_4$ nanoparticles. Using this $Fe_3O_4$ seed-mediated growth method, $Fe_3O_4$ nanoparticles with larger size (up to 20 nm) could be synthesized.

2.1.4. Surface modification of $Fe_3O_4$ nanoparticle core

Under atmospheric conditions, 200 $\mu$l of n-hexane solvent with 20 mg of dispersed hydrophobic $Fe_3O_4$ nanoparticle core was added to 2 ml of dichloromethane suspension containing 20 mg of tetramethylammonium salt of 11-aminoundecanoic acid. The mixture was shaken for 20 minutes, while a magnet was used to separate the precipitated $Fe_3O_4$ nanoparticles. The solvent and the non-magnetic suspended matter were decanted, the resulting precipitate was washed once with dichloromethane, and then the separation with magnet was performed again to remove excess surfactant. The resulting product was dried under nitrogen gas and then dispersed in deionized water or pH-neutral PBS.

2.2 Hydrothermal synthesis method

1.35 g (5 mmol) of ferric chloride hexahydrate ($FeCl_3 \cdot 6H_2O$) was dissolved in 40 mL of ethylene glycol to form a clear solution. To the above solution, 3.6 g of sodium acetate and 1.0 g of polyethylene glycol were added, stirred vigorously for 30 minutes, and then transferred to a 50 ml sealed stainless steel autoclave, reacted at 200 °C for 8-72 hours, and then cooled to room temperature. The black product obtained in the reaction was washed with ethanol for several times and then dried at 60 °C for 6 hours, to obtain a magnetic nanoparticle core having a particle diameter of 10 nm or less.

2.3 Microemulsion method (reverse micellar method)

5 mmol of $Mn(NO_3)_3$ and 10 mmol of $Fe(NO_3)_3$ were dissolved in 25 mL of deionized water to form a clear and transparent solution; 25 mL of 0.4 M NaDBS ($[CH_3(CH_2)_{11}(C_6H_4)SO_3]Na$) was added to the above iron ion solution, and then added with a large volume of toluene, in which the size of the resulting $MnFe_2O_4$ nanoparticles particles depended on the volume ratio of water and toluene. For example, in order to obtain 8nm nanoparticles, the volume ratio of water and toluene should be 5 : 100. After the above mixture liquid was stirred overnight, it became a clear single-phase solution containing reversed micelles.

In order to form colloids in the reversed micelles, 40 mL of 1 M NaOH solution was added dropwise with vigorous stirring, and the stirring was continued for 2 hours. The water and most of the toluene in the solution were removed by distillation to reduce the volume of the solution. The resulting concentrated solution containing suspended colloids was washed with water and ethanol to remove excess surfactant in the solution. A primary magnetic nanoparticle core was obtained by ultracentrifugation, and a nanocrystal was obtained by heating at 350 °C under nitrogen atmosphere for 12 hours.

Example 2: Hydrophobic polystyrene surface modification

(Modification on the magnetic nanoparticle core ($MnFe_2O_4$) obtained in Example 1)

$MnFe_2O_4$ nanoparticles with an average particle size of 9 nm were added to an aqueous solution/3-chloropropionic acid solution with a concentration of 1.0 mol / L initiator, the solution was adjusted to pH of 4 with hydrochloric acid, and stirred overnight. The nanoparticles were collected with a magnet, washed with water for several times to remove excess 3-chloropropionic acid. 0.22 g of dried nanoparticles were added to 8 mL of polystyrene solution under continuous feeding of nitrogen gas, followed by the addition of 4 mL of an xylene solution of 0.3 mmol of CuCl and 1.1 mmol of 4,4'-dinonyl-2,2-dipyridine. The above mixture reacted at 130 °C for 24 h under continuous agitation. The nanoparticles were collected with a magnet and washed repeatedly with toluene to obtain polystyrene-coated magnetic iron oxide nanoparticles.

Example 3: Hydrophilic polyacrylic acid modification

1 g of $Fe_3O_4$ obtained in Example 1 (for example, 10 nm $Fe_3O_4$ obtained by co-precipitation method) and 5 ml of a silane coupling agent (methacryloxypropyltrimethoxysilane, KH570) were mixed with 50 ml of xylene in a reaction flask. Under nitrogen protection, the reaction was carried out under stirring at 80 °C for 3 hours. After the reaction, the mixture was centrifuged and washed with ethanol three times to remove the silane coupling agent adsorbed on the surface of the $Fe_3O_4$, and vacuum-dried for 12 hours. The above-mentioned silane coupling agent-activated $Fe_3O_4$, 40 mg of potassium persulfate and 30 ml of deionized water were added in a flask, reacted under under nitrogen protection and stirring at 40 °C for 10 minutes. Then, 4ml of acrylic acid was slowly dropped into the flask, and reacted under nitrogen protection and stirring at 40 °C for 1 hour. The nanoparticles were magnetically separated, washed three times with deionized water and finally dried under vacuum.

Example 4: Photosensitive functional modification of nanoparticles

1. Synthesis of photosensitive functional monomer

The synthesis method of photosensitive monomer with photo-crosslinking characteristics comprised: 4-vinylbenzophenone (4VBP) and styrene monomer were directly polymerized by atom transfer radical polymerization (ATRP) to obtain a photosensitive polystyrene-polyvinylbenzophenone copolymer (PS-PVBP), wherein the specific steps were as follows: in a dry Schlenk tube connected with a reflux condenser, Cu(I)Br (0.695 mg, 4.8 umol), 4VBP (1.0 g, 4.8 mmol), styrene (2u, 20 umol) and 4-vinylbenzophenone (1 $\mu$L, 4.8 umol) were added, and the mixture was degassed for three times by means of freeze-pump-thaw circulation. Methyl bromopropionate (5.35 $\mu$L, 48 umol) was added to the above mixture at -78 °C under condition of nitrogen with positive pressure, and the mixture was degassed again for three more times by means of freeze-pump-thaw circulation. Polymerization was carried out by heating the mixture to a temperature of 85 °C under negative pressure, and the reaction was allowed to proceed for 4 hours. The above Schlenk tube was immersed in liquid nitrogen, and 10 ml of dichloromethane was added to dissolve the polymer. The resulting solution was precipitated twice with methanol ($2 \times 300$ mL) to give $PS_x$-$PVBP_y$ as a light yellow solid, wherein x: y ~ (60% -90%), and a preferred composition was $PS_{75}$-$PVBP_{25}$. In the same way, $PS_{75}$-$PVBP_{25}$-$PAA_{100}$ could be obtained by adding hydrophilic monomer acrylic acid.

2. Preparation of nanoparticles surrounded with photosensitive cross-linked micelles

(Polystyrene$_{75}$-co-polyvinylbenzophenone$_{25}$)-polyacrylic acid$_{100}$, namely $(PS_{75}$-co-$PVBP_{25})_{115}$-b-$PAA_{100}$, was chosen as a polymer to form micelles in aqueous solution. 5 mg of $(PS_{75}$-co-$PVBP_{25})_{115}$-b-$PAA_{100}$ and 10 mg of $Fe_3O_4$ obtained in Example 1 (for example, 10 nm $Fe_3O_4$ obtained by co-precipitation method) were dissolved in 10 ml of dimethylformamide (DMF) solution; and then double distilled water (0.1 ml / min) was gradually added under vigorous stirring. When the volume of water reached 60%, the resulting solution was added to a dialysis membrane with a molecular weight cut off of 12K-14K and dialyzed against water for 24 hour to remove DMF, and then the micellar solution was transferred to a quartz tube and irradiated with a laser at different time periods (emission wavelength: 315-400 nm) to form a photosensitive monomer-coated nanoparticle.

Example 5: Thermosensitive functional modification of nanoparticles

1 g of $Fe_3O_4$ obtained in Example 1 (for example, 10 nm $Fe_3O_4$ obtained by co-precipitation method) and 5 ml of a silane coupling agent (methacryloxypropyltrimethoxysilane, KH570) were mixed with 50 ml of xylene in a reaction flask. Under nitrogen protection, the reaction was carried out with stirring at 80 °C for 3 hours. After the completion of the reaction, the mixture was centrifuged and washed with ethanol three times to remove the silane coupling agent adsorbed on the surface of the $Fe_3O_4$, and vacuum-dried for 12 hours. The above-mentioned silane coupling agent-activated $Fe_3O_4$, 40 mg of potassium persulfate and 30 ml of deionized water were added in a flask, reacted under nitrogen protection and stirring at 40 °C for 10 minutes. Then, 4ml of N-isopropylacrylamide aqueous solution was slowly dropped into the flask, and reacted under nitrogen protection and stirring at 40 °C for 1 hour. The nanoparticles were magnetically separated, washed three times with deionized water and finally dried in vacuo.

Example 6: pH-Sensitive functional modifications of nanoparticles

1 g of $Fe_3O_4$ obtained in Example 1 (for example, 10 nm $Fe_3O_4$ obtained by co-precipitation method) and 5 ml of a silane coupling agent (methacryloxypropyltrimethoxysilane, KH570) were mixed with 50 ml of xylene in a reaction flask, and reacted under nitrogen protection and stirring at 80 °C for 3 hours. After the completion of the reaction, the mixture was centrifuged and washed with ethanol three times to remove the silane coupling agent adsorbed on the surface of the $Fe_3O_4$, and dried in vacuo for 12 hours. The above-mentioned silane coupling agent-activated $Fe_3O_4$, 40 mg of potassium persulfate and 30 ml of deionized water were added in a flask, reacted under nitrogen protection and stirring at 40 °C for 10 minutes. Then, 4ml of dimethyl amino ethyl methacrylate aqueous solution was slowly dropped into the flask, and reacted at 40 °C for 1 hour under stirring and nitrogen protection. The nanoparticles were magnetically separated, washed three times with deionized water and finally dried in vacuo.

Example 7: Biocompatibility evaluation

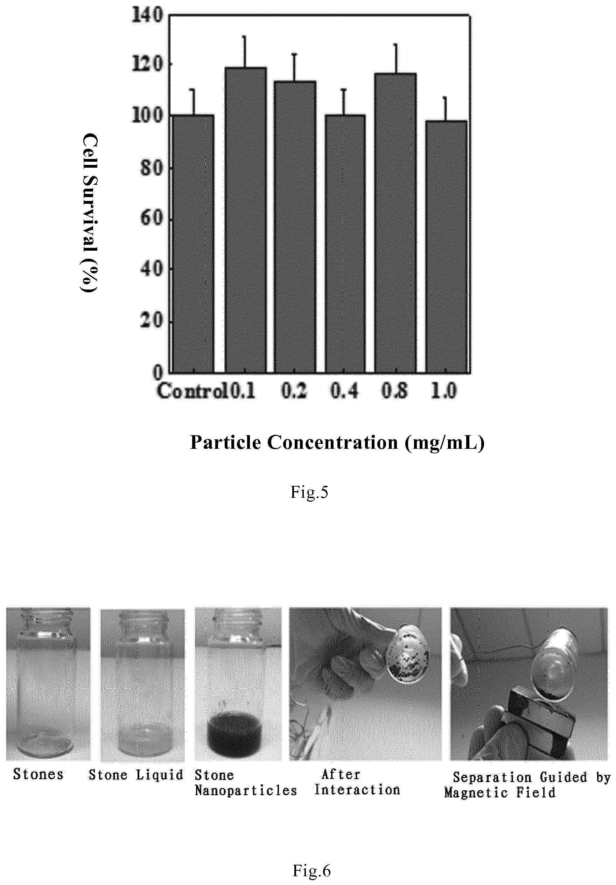

Cell Plating: 293t cells in logarithmic growth phase were digested, counted after centrifugation, and plated on a 96-well plate with a cell density of $5.0\times10^4$/well. 100 $\mu$l of serum-containing medium was added to each well. The surronding blank wells were each complemented with 100 $\mu$l of serum-containing medium. The plate was placed in a 7% $CO_2$, 37 °C cell incubator overnight. The hydrophilically modified magnetic nanoparticles of Example 3 in an amount of 100 $\mu$l per well were added to the cell wells and incubated with N87 cells, wherein the magnetic nanoparticles obtained in Example 3 was used in concentrations of 0.1, 0.2, 0.4, 0.8, 1.0 mg/ml respectively. After incubated at 37 °C for 24 hours, the cells were gently washed twice with culture medium, and then the cell viability was measured with a Cell Counting Kit-8 kit, in which the detection conditions were as follows: 10 $\mu$l of CCK-8 reagent per well, incubate at 37 °C for 2 hours, read the absorbance value at 450nm with BIO-TEK ELx800 automatic microplate reader, and calculate the cell viability. The results shown in Figure 5, indicating the synthesized hydrophilically modified magnetic nanoparticles had good biocompatibility and almost no toxicity in vivo, and giving preliminary evidences of applicability for in vivo experiments.

Example 8: Evaluation of in vitro stone separation with assistance of nanoparticles

As shown in Fig.6, a certain amount of stones were weighed, pulverized with a pestle into a powder (particle size 0.5-2mm), poured into a transparent glass bottle, and PBS solution was added to obtain a stone liquid. After being mixed uniformly, the hydrophilically modified magnetic particles of Example 3 at a concentration of 1 mg / ml was used as a separation liquid and added, and then gently shaken. After standing for 5 minutes, separation was carried out with a magnet. During standing, it was observed that the color of the mixture gradually faded, and after 5 minutes, it was observed that the black magnetic particles were adsorbed on the surface of the stone. Under the guidance of the magnetic field, the stones with magnetic particles adsorbed on their surface moved toward the magnet.

Example 9: Evaluation of in vivo safety of nanoparticles in animals

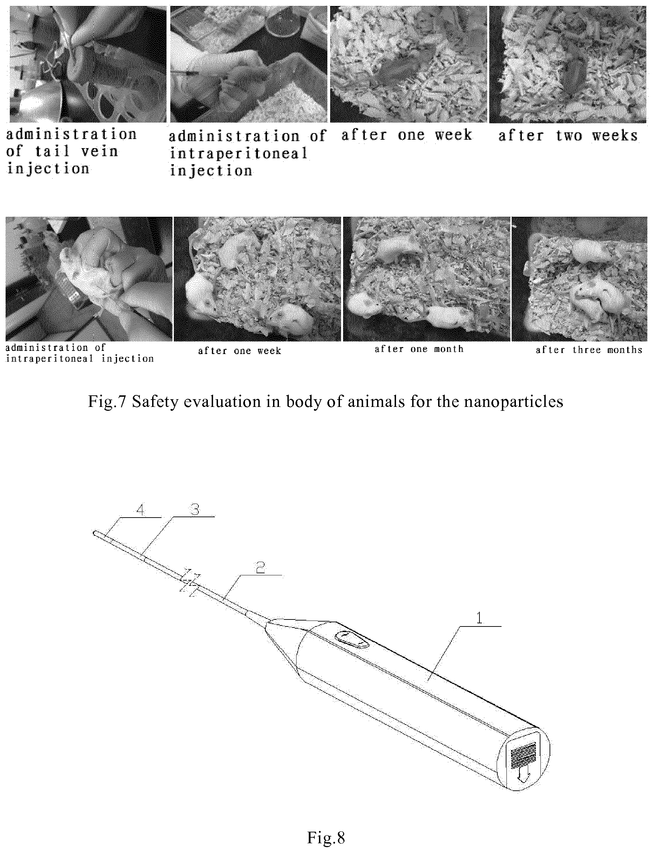

As shown in Fig.7, one 6-week-old nude rat and 3 mice were intraperitoneally injected and tail vein injected, respectively. The hydrophilically modified magnetic nanoparticles of Example 3 at a concentration of 0.5 mg / ml and in an amount of 200 $\mu$L were used for the injection for consecutive two days. The living conditions of the mice were observed periodically (such as one week, two weeks, etc.). The results showed that after intravenous injection of 200$\mu$L of magnetic nanoparticle solution, no obvious toxicity was found in the nude rat and the mice within 1 week; and after intraperitoneal injection of particle solution at the same concentration 3 times, it was found that the rat and the mice had good survival status within 3 months. This shows that the prepared magnetic nanoparticles have good biocompatibility, and such preliminary assessment shows they basically have no acute toxicity and chronic toxicity.

Example 10: Using magnetic nanoparticles in removal of urinary stones

In the treatment of disease of kidney stones, an ureteroscope was used for holmium laser lithotripsy. In the operation, after crushing process of kidney stones with holmium laser, 200ml of the hydrophilic magnetic nanoparticle solution of Example 3 according to the present invention was injected into the kidney through the working channel of the ureteroscope, so that the hydrophilic magnetic nanoparticle solution was thoroughly mixed with the stone debris. After about 3 minutes, the nanoparticles in the solution completely adhered to the surface of the stone debris, and magnetized the stone debris. The above-mentioned magnetic probe rod system for removal of stones in urinary system, in which a NdFeB permanent magnet was used as the magnetic field source 3, was inserted into the kidney through the working channel of the ureteroscope. Under the action of the magnetic field at the distal end of the magnetic probe rod system, the magnetized stone debris moved closer to the distal end of the magnetic probe rod and attracted to the magnetic probe rod. Finally, the magnetic probe rod system filled with stones debris at its distal end together with the ureteroscope were drawn from the body through the soft sheath of the ureteroscope. After the the stone debris were removed from the distal end of the magnetic probe rod, the magnetic probe rod could be inserted again into the endoscope to carry out stone removal operation, until all of the stone debris inside the kidney were removed from the body. In this Example, the stone had a diameter of about 20 mm and was located in the lower kidney calyx. By using the magnetic probe rod system in combination with the hydrophilic magnetic nanoparticle material of the present invention, all (100%) of the stone debris were removed from the body. In comparison with the traditional method of removing stones by baskets and the method of pulverizing stones and then allowing patients to discharge stone debris, the technical solutions of the present invention achieved simple, efficient and complete removal of stones, thereby greatly improving the efficiency and safety of stone removal operations.

Example 11: Comparison of different methods for stone extraction

Step 1. Synthesis of magnetic particles

From the left mouth of the three-mouth bottle, ferrous iron and ferric iron solutions were added, the total volume was 200ml. The left mouth was sealed with a glass stopper, the right mouth was sealed with nitrogen gas, and the mechanical stirrer was set at a speed of 500, timing for 30min. After 30min, aqueous ammonia was added dropwise with a syringe to adjust pH to 10.0; and timing for 30 min again; a water bath at 80 °C was prepared, and the three-mouth bottle was transferred to the water bath, and the reaction was continued for 30 minutes. After 30 minutes, the instrument was turned off and allowed to cool to room temperature. After being washed with deionized water twice, acetone once, the product was placed in an oven for drying.

Step 2. Screening

After being dried, the product was ground, ans screened over mesh sieves into three grades of magnetic particles: $d \leq 0.048mm$, $0.048mm \leq d \leq 0.106mm$, $d \geq 0.106mm$.

Step 3. Surface modification of magnetic particles

150mg of magnetic particles was added in a three-necked flask into which a magnetic rotor was placed. When oil bath temperature was elevated to 80 degrees Celsius, 15ml of xylene solution was added, fed with nitrogen gas immediately and sealed, adjusting the rotation speed. After 10 minutes, 1.5 ml of silane was added, timing for 6 hours, and a nitrogen pack was connected; After 6 hours, the three-necked flask was removed, the xylene was poured out, the product was washed with ethanol twice, 10 minutes for each time, 20ml for each dose, and then washed with dd water twice. 15ml of deionized water was prepared, the magnetic particles was transferred to the bottle, fed with nitrogen gas for 20 minutes, adjusting the rotation speed at 3 and the oil bath temperature of 70 °C. 20mg of potassium persulfate dissolved in a solution at room temperature was added, continuously passing nitrogen gas for 10 minutes. 1.5ml of acrylic acid was added dropwise, the reaction in the flask was observed; adjusting rotation speed, and stopping the reaction after 20 minutes. The product was washed with sodium hydroxide solution twice, transferred to a beaker, and then washed twice with deionized water; re-suspended in water.

In order to compare the stone removal performance of the lithotomy with magnetic material (i.e., magnetic nanoparticles) of the present application with the polymer gel stone removal method existing in the prior art, the inventor designed a stone basket, and a cationic polymer polyethylenimine gel with gel composition according to CN105283140A was used to simulate the gel in the kit.

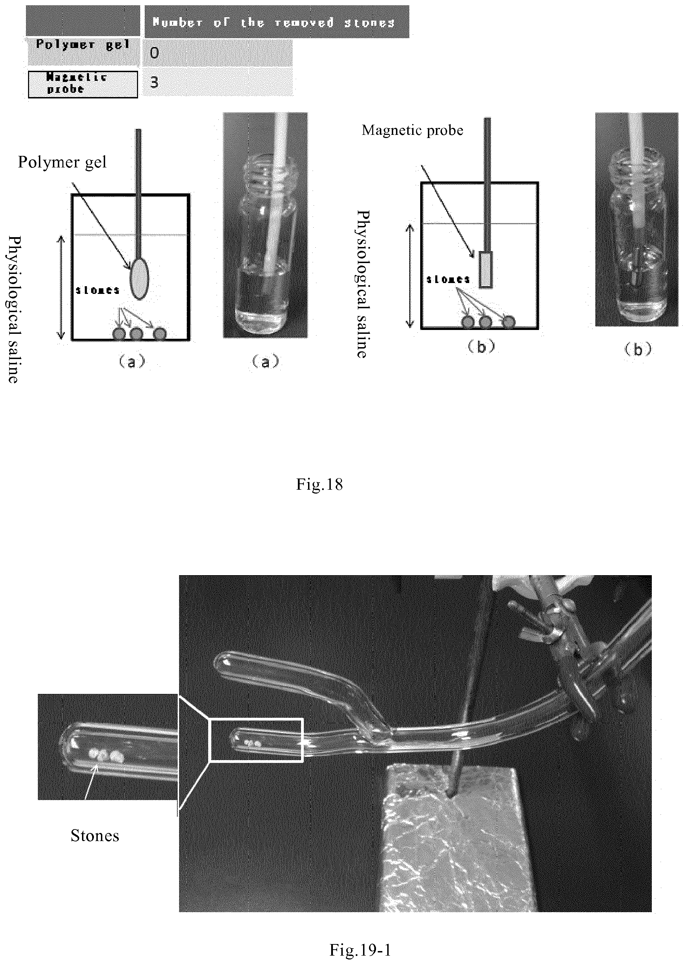

Specifically, two glass bottles as shown in Fig.18 were used, and each of them was added with 3 pieces of 2-3 mm blank stones, the stones in the bottle (a) were not modified, while the stones in the bottle (b) were treated with 1 ml (3 mg / ml) of the magnetic particle solution of step 3 for modification. After 5 minutes, the magnetic particle solution was sucked out from the bottle (b), and then the bottles (a) and (b) were added with 2ml of physiological saline, respectively.

The experimental results show that the magnetic particles had stone removal effect (3 stones were removed) significantly superior to the polymer gel stone removal method (0 stone was removed).

From Example 11, it can be seen that the magnetic nanoparticles as claimed in the present invention have excellent effect in stone removal applications relative to the gel stone removal method existing in the prior art (for example, CN105283140A).

Example 12: Effects of two mimicking experiments for stone removal in vivo

The purpose of this experiment was to simulate stone removal effects gradually and really from simple to complex. Therefore, the experiment was divided into two parts:

1. Using an urethra model with two calices simulated with transparent glass tube

Modification of stones: To simulate the in vivo environment, three stones with diameter of 2-3 mm were placed in the transparent glass tube for simulating urethra model with two calices, which was the initial state of the environment for the stone in kidney (see Fig.19-1).

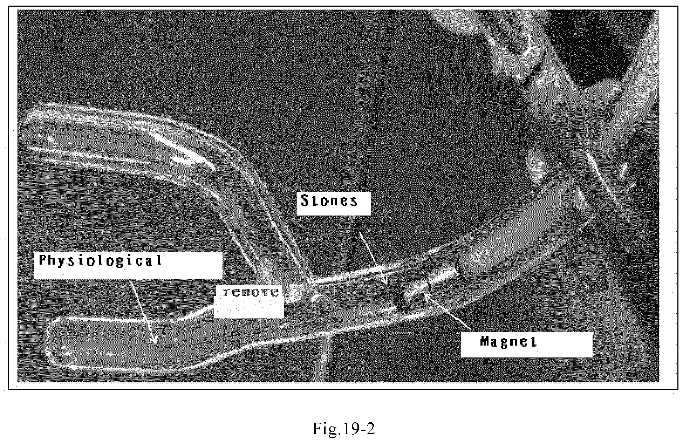

The whole chamber was filled with physiological saline, and 1 ml (3 mg / ml) of the magnetic particle solution (the magnetic particles obtained in Example 11) was injected through a microinjector catheter, timing for 5 min. The experimental results showed that when a magnetic probe was used to verify stone removal effect, the stone could be quick guided and removed successfully (see Fig. 19-2).

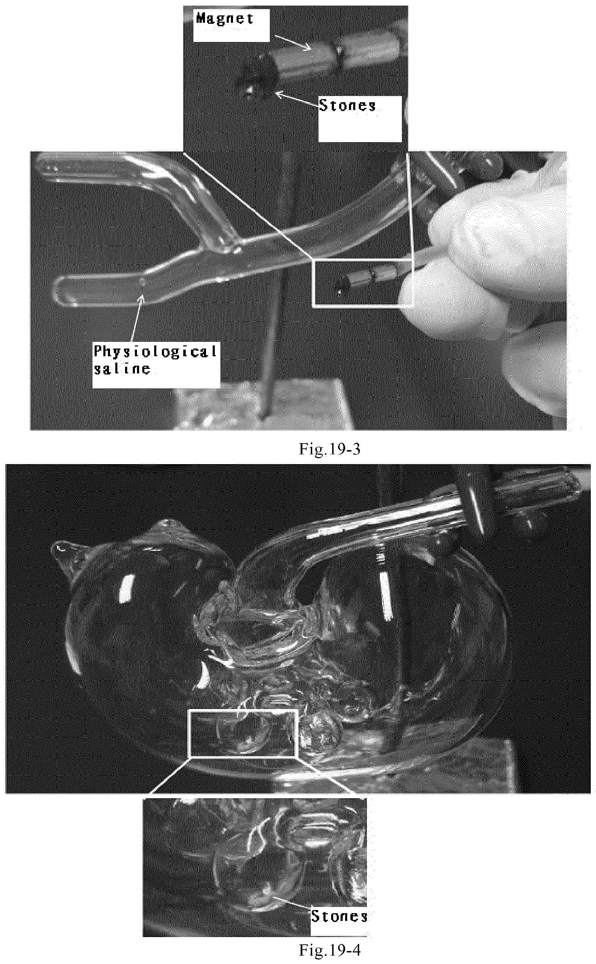

The results of the adsorption of stones on the magnetic probe were shown in Fig.19-3.

2. Using a transparent artificial kidney model

Compared with the previous model, this model was basically the same in structure, size and shape as a human kidney, and had more complicated structure and pipeline obstructions. The purpose was to perform further in vitro simulation, and to observe the effects of obstructions possibly existing in kidney calices and urethra on stone removal.

Modification of stones: In order to better simulate the environment for stones in kidney, a glass model was made, and three stones with diameter of 2-3 mm were placed in the transparent artificial kidney model, which was the initial state of the environment for stones in kidney (see Fig.19 -4).

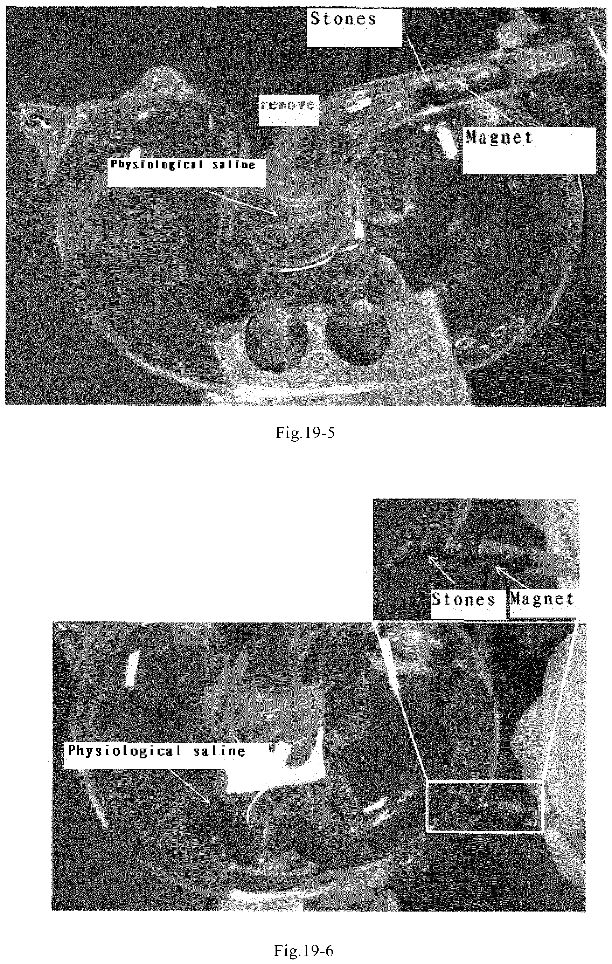

The whole chamber was filled with physiological saline, and 1 ml (3 mg / ml) of the magnetic particle solution (the magnetic particles obtained in Example 11) was injected through a microinjector catheter, timing for 5min. The experimental results showed that when a magnetic probe was used to verify stone removal effect, the obstructions in calices and pipeline basically had not effect on the removal of stone, and the stones were removed successfully (see Fig.19-5).

The results of the adsorption of stones on the magnetic probe were shown in Fig.19-6.

From Example 12, it can be seen that the magnetic nanoparticles of the present application have excellent effects in the two experiments that simulate the removal of stones in vivo, and furthermore, the magnetic nanoparticles have a good application prospect in stone removal applications.

While many embodiments of the invention have been shown and / or discussed herein, it is not intended that the invention is limited thereto. It is anticipated that the scope of the present invention will be the same as what is allowed in the art and interpreted from the description. Therefore, the above description should not be construed as limitation, but merely as exemplifications of specific embodiments. Those skilled in the art can envision other variations within the scope and spirit of Claims appended hereto.

## Claims

1. A use of a magnetic material in removal of a stone, wherein the magnetic material magnetizes the stone by physical adsorption, chemical bonding or the like, and removes the stone by the action of a non-contact magnetic field.

2. The use according to Claim 1, wherein the magnetic material comprises the following components: a preparation containing a magnetic metal element or a compound thereof; and materials capable of binding to calcium salt.

3. The use according to Claim 2, wherein the preparation containing the magnetic metal element or compound thereof and the materials capable of binding to calcium salt form a structure that may be a clad structure or a core-shell structure, for example, the materials are completely or partially covered on the surface of the preparation; or form a modified structure in which the materials are bonded to the surface of the preparation by absorption; or form a complex structure in which the preparation and the materials form a physical mixture; or form a composite structure of the aforementioned structures.

4. The use according to Claim 2, wherein the preparation containing the magnetic metal element or compound thereof is in nano-scale or in micro-scale.

5. The use according to Claim 2, wherein the materials capable of binding to calcium salt are surfactants or polymer compounds.

6. The use according to Claim 2, wherein the materials capable of binding to calcium salt are macromolecular compounds.

7. The use according to Claim 1, wherein the magnetic material includes carboxyl, amido, amino, mercapto, hydroxyl, carbonyl, ether group, amine group, ester group, carbamate group, carbamido or quaternary amine group, sulfonic acid group, sulfhydryl, phosphine group or conjugate acid or base thereof, epoxy group, chlorine group, sulfate group, phosphinic acid, sulfinic acid, carboxylic anhydride group, hydrosilyl group, amine group and moiety of any combination thereof, aldehyde group, unsaturated double bond, phosphoric acid group, halogen group, N-succinimido, maleimido, ethylenediaminetriacetic acid alkyl group, polyethylene glycol, polyamino acid or glycan, preferably carboxyl, amido, mercapto, carbamate group, carbamido, sulfonic acid group, phosphino conjugate acid, phosphino basic group, sulfate group, phosphinic acid, sulfinic acid, N-succinimido, maleimido, polyamino acid.

8. The use according to Claim 1, wherein the magnetic material is in the shape of bar, line, band, sheet, tube, pomegranate, cube, three-dimensional flower, petal, chestnut, four-pointed star, shuttle, rice grain, sea urchin, chain ball, rugby ball, string of beads, snowflake, ellipsoid, sphere, regular tetrahedron, regular hexahedron, regular octahedron, quasi-sphere, popcorn, cross, strip, rod, cone, disc, branch, web, simple cubic, body-centered cubic, face-centered cubic, simple tetragon, body-centered tetragon, simple orthogon, body-centered orthogon, single-face-centered orthogon, multi-shell, laminar, preferably the shape of sphere, quasi-sphere, pomegranate, chestnut, sea urchin, chain ball, string of beads.

9. The use according to Claim 1, wherein the magnetic material includes a magnetic fluid, a magnetic liposome, a magnetic microcapsule, a magnetic microsphere, a magnetic emulsion, a magnetic nanoparticle, a magnetic nanotube, a magnetic nanowire, a magnetic nanorod, a magnetic nanoribbons, preferably a magnetic fluid, a magnetic liposome, a magnetic microsphere, a magnetic nanotube.

10. The use according to Claim 9, wherein the magnetic liposome includes a magnetic liposome which surface is modified to carry a functional group as described in Claim 7.

11. The use according to Claim 9, wherein the type of the magnetic liposome includes a single layer, a multi-layer, a multi-vesicle, and the preparation method of the magnetic liposome includes preferably a film dispersion method and an ultrasonic dispersion method.

12. The use according to Claim 9, wherein the magnetic fluid is a stable suspension liquid composed of magnetic particles, a carrier liquid (mineral oil, silicone oil, etc.) and a surfactant, the magnetic particles that can be used for removal of stones comprise magnetic nanoparticles which surface is modified with the functional group according to Claim 7, and also comprise a surfactant with the functional group as described in Claim 7.

13. The use according to Claim 9, wherein the preparation method for the magnetic fluid includes a chemical co-precipitation method, a sol-gel method, a hydrothermal synthesis method, a microemulsion method, a phase transfer method, preferably a co-precipitation method, a sol-gel method, a hydrothermal synthesis method.

14. The use according to Claim 9, wherein the magnetic microsphere is **characterized by** a surface modified with or covered with the functional group as described in Claim 7.

15. The use according to Claim 9, wherein the preparation method for the magnetic microsphere includes an emulsion volatilization method, a solvent replacement method and a salting-out method.

16. The use according to Claim 1, the magnetic nanotube comprises a magnetic nanotube in which a magnetic material is filled in the tube and also a magnetic nanotube in which a magnetic material covers outside the tube, the surface of which has the functional group described in Claim 7, and the functional group may be derived from the magnetic material covered on surface or from the nanotube itself.

17. The use according to Claim 1, wherein the preparation method for the magnetic nanotube includes a chemical vapor deposition method, a co-precipitation method, a dip-pyrolysis method, an electroless plating method and a self-assembly method, and the preferred method is a co-precipitation method.

18. The use according to Claim 1, the magnetic nanoparticle is surface-modified or covered with the functional group as described in Claim 7.

19. The use according to Claim 1, wherein the magnetic material constitutes a nanoparticle core; and the nanoparticle core is in-situ modified with a surface modifier monomer by using an initiator and / or a crosslinking agent to form a nanoparticle shell.

20. The use according to Claim 19, wherein the nanoparticle core has a diameter of 2-50 nm, and a weight percentage of 30-95% relative to the whole weight of the nanoparticle, and its magnetic material includes a compound of $Fe^{3+}$, $Fe^{2+}$, $Mn^{2+}$ or $Ni^{2+}$, or a metal element selected from iron, nickel, copper, cobalt, platinum, gold, europium, gadolinium, dysprosium, terbium, or a composite or oxide of the aforementioned metals, or any one of the above items or a combination of two or more of the above items, preferably a compound of $Fe^{3+}$, $Fe^{2+}$, $Mn^{2+}$ or $Ni^{2+}$, more more preferably $Fe^{3+}$ and $Fe^{2+}$ in a ratio of 15% to 85%, preferably 1: 2.5 to 1.5: 1.

21. The use according to Claim 19, wherein the mutual forces for surrounding and crosslinking between the nanoparticle and stone include van der Waals force, hydrophobic interaction, adsorption and surface deposition that form surrounding interaction; a chemical bond formed between carboxyl-stone, including a hydrogen bond, an ester bond, an amide bond and other covalent bonds; physical and chemical inter-chain entanglements between chains and chemical crosslinking between chains.

22. The use according to any one of Claims 19-22, wherein the surface modifier includes a hydrophilic surface modifier with function response, a hydrophobic surface modifier with function response, a photosensitive surface modifier with function response, a thermosensitive surface modifier with function response or a pH sensitive surface modifier with function response, wherein the hydrophilic surface modifier includes acrylic acid, methacrylic acid, isobutyl acrylamide or poly N-substituted isopropylacrylamide; the hydrophobic surface modifier includes olefins, preferably polystyrene, polyethylene or oleic acid; the photosensitive surface modifier is selected from the group consisting of azos and quinolines and benzophenones (PVBP), preferably ethylene benzophenone; the thermosensitive surface modifier is selected from amphiphilic polymers with amide bond, preferably polyacrylamide or poly N-substituted isopropylacrylamide; the pH-sensitive surface modifier is selected from the group consisting of polymers with carboxyl group and quaternary ammonium salt, preferably a polyacrylic acid, dimethyl amino ethyl ester and dimethylaminopropyl methacrylate; the shell accounts for 2-40% by weight of the nano-particle, preferably the particle is of a shape of sphere, rod or diamond.

23. The use according to any one of Claims 19 to 22, wherein the crosslinking agent includes 3-(methacryloyloxy)propyltriethoxysilane, divinylbenzene, diisocyanate or N,N-methylenebisacrylamide, and the initiator includes 3-chloropropionic acid, CuCl, 4,4'-dinonyl-2,2-bipyridine or potassium persulfate.

24. The use according to any one of Claims 19-22, wherein the preparation method for the nanoparticle includes the steps of:

a) preparing the nanoparticle core using the magnetic material;
b) forming the nanoparticle by in situ linking the surface modifier monomer to the nanoparticle core by the initiator and / or crosslinking agent to form the nanoparticle shell.

25. The use according to Claim 24, wherein the magnetic material includes a compound of $Fe^{3+}$, $Fe^{2+}$, $Mn^{2+}$ or $Ni^{2+}$, or a metal element selected from iron, nickel, copper, cobalt, platinum, gold, europium, gadolinium, dysprosium, terbium, or a composite or oxide of the aforementioned metals, or any one of the above items or a combination of two or more of the above items, preferably $Fe_3O_4$, $MnFe_2O_4$, $\gamma$-$Fe_2O_3$ or other nanoscale-sized ferrite particles, more preferably $FeCl_3 \cdot 6H_2O$ and $FeCl_2 \cdot 4H_2O$ in a molar ratio of 15% to 85%, preferably 1:2.5 to 1.5:1, and is prepared by the following steps:

dissolving a proportion of the metal salt-containing material in water;
feeding nitrogen to expel oxygen in the solution;
adding a catalyst at a room temperature of 10-40°C, preferably 30°C to adjust the pH to 7-12, preferably 10; keeping agitation for 10-60 minutes; and
reacting under condition of 40-100°C, preferably 70°C water bath, for 20-40 minutes, then separating with a magnet and drying to obtain the magnetic nanoparticle core.

26. The use according to Claim 25, wherein when aqueous ammonia is used as the catalyst for preparing the nanoparticle, the method for adding aqueous ammonia is a continuous dropping method with assistance of an electronic pump at a speed of 20-100 drops / minute, preferably 40-60 drops / minute; and when the magnetic material is a liquid monomer material, the liquid monomer is added dropwise in continuous manner with assistance of an electronic pump, and the reaction agitation is at a speed of 100-1000 revolutions / minute, preferably 500-700 revolutions / minute.

27. The use according to any one of Claims 25-27, wherein said use further includes performing hydrophobic surface modification on the obtained nanoparticle core, comprising the steps of:

dispersing the prepared nanoparticle core in an aqueous solution and added with a xylene solution of 3-chloropropionic acid, polystyrene, CuCl and 4,4'-dinonyl-2,2-dipyridine, and the molar ration between the above-mentioned nanoparticle core and the reaction solution is 1:1;

reacting the above mixture at 130°C with continuous agitation for 15-30h, preferably for 24 hours; and

collecting the nanoparticle with a magnet and washing repeatedly with toluene to obtain a hydrophobic polystyrene-coated magnetic nanoparticle.

28. The use according to any one of Claims 25-27, wherein said use further includes performing hydrophilic surface modification on the obtained nanoparticle core comprising the steps of:

dispersing the nanoparticle core in xylene, and adding a silane coupling agent, wherein the ratio of the added nanoparticles, xylene and silane coupling agent is 95: 5;

reacting under protection of nitrogen atmosphere at a temperature of 20 to 100 °C, preferably 80 °C for 2 to 5 hours, preferably 3 hours;

washing with an alcohol solvent and drying for 12h, dispersing in an aqueous solution under ultrasonic condition, adding with potassium persulfate;

reacting under protection of nitrogen atmosphere at 40-80 °C for 10 minutes, then adding with acrylic acid and continuously reacting at 40-80 °C for 1 hour, preferably reacting at 70 °C; and

separating by a magnet, washing and drying to prepare and obtain a polyacrylic acid-modified hydrophilic nanoparticle.

29. The use according to any one of Claims 25-27, wherein said use further includes performing a photosensitive, thermosensitive or pH-sensitive surface modification based on the resulting nanoparticle core or hydrophilic surface, or a hydrophilic, hydrophobic, photosensitive, thermosensitive and pH-sensitive co-modification based on the resulting nanoparticle core, wherein the re-modification on the hydrophilic surface includes the steps of:
dissolving and dispersing the polyacrylic acid-modified magnetic nanoparticles in an alcoholic solvent, adding with a photosensitive monomer such as ethylene benzophenone, a thermosensitive monomer such as N-isopropylacrylamide, or a pH-sensitive monomer such as dimethylaminopropyl methacrylate or a blend monomer of acrylic acid and styrene, keeping reaction at 40-80 °C for 1 h, preferably at a reaction temperature of 70 °C; and separating with a magnet, washing and drying to obtain a photosensitive, thermosensitive or pH sensitive functional monomer-modified magnetic nanoparticles, respectively.

30. The use according to Claim 1, wherein the stone includes urinary system stones, such as kidney stones, ureteral stones and bladder stones, human biliary system stones, and stone-like particles in other organs.

31. The use according to Claim 1, wherein the interactions between the magnetic material and stone include ionic bonds, van der Waals forces that form surrounding interactions, hydrophobic interactions, adsorption and surface deposition; chemical bonds between the carboxyl-stone, including hydrogen bonds, ester bonds, amide bonds and other covalent bonds; physical and chemical inter-chain entanglement between chains and chemical crosslinking between chains.

Fig.1 (Upper, left: sphere shape; middle: rod shape; right: diamond shape; Lower, particle size distribution)

Fig.2 (Left: Particle size distribution diagram; Right: TEM diagram)

Fig.3

Fig.4

Fig.5

Stones   Stone Liquid  Stone        After        Separation Guided by
Nanoparticles  Interaction     Magnetic Field

Fig.6

administration
of tail vein
injection

administration of
intraperitoneal
injection

after one week

after two weeks

administration of
intraperitoneal injection

after one week

after one month

after three months

Fig.7 Safety evaluation in body of animals for the nanoparticles

Fig.8

1

12a

13a

Fig. 9

11

1

12b

Fig.10

Fig. 11

Fig.12

Fig.13

Fig. 14

Mutual force $F_{(mutual\ force)}$ caused by different action modes

Surrounding between particles-stone caused by different mutual forces

Entanglement and crosslinking between particles caused by magnetic particles

Including van der Waal's force, covalent bonds formed between carboxyl and stone, physical entanglement between chains and chemical crosslinking

Stone

Superparamagnetic nanoparticle

$S$ hydrophobic surface

$D$ stone

$R$ magnetic particle

$S$ particle surface properties

Diagram of the principle of interaction between magnetic particles and stones

Fig.15

Fig.16

Fig.17

| | Number of the removed stones |
|---|---|
| Polymer gel | 0 |
| Magnetic probe | 3 |

Polymer gel

Physiological saline

(a)  (a)

Magnetic probe

Physiological saline

(b)  (b)

Fig.18

Stones

Fig.19-1

Fig.19-2

Fig.19-3

Fig.19-4

Fig.19-5

Fig.19-6

# INTERNATIONAL SEARCH REPORT

| International application No. |
| --- |
| **PCT/CN2016/100804** |

### A. CLASSIFICATION OF SUBJECT MATTER

A61L 31/02 (2006.01) i; A61B 17/22 (2006.01) i

According to International Patent Classification (IPC) or to both national classification and IPC

### B. FIELDS SEARCHED

Minimum documentation searched (classification system followed by classification symbols)

A61L 31/-, A61B 17/-

Documentation searched other than minimum documentation to the extent that such documents are included in the fields searched

Electronic data base consulted during the international search (name of data base and, where practicable, search terms used)

CNABS, CNTXT, CNKI, WPI, EPODOC, WANFANG: SHANGHAI KELI'ANQIN TECHNOLOGY DEVELOPMENT CO., LTD.;

SUN, Yinghao; stone, magnet+, kidney stone?, nano particle?

### C. DOCUMENTS CONSIDERED TO BE RELEVANT

| Category* | Citation of document, with indication, where appropriate, of the relevant passages | Relevant to claim No. |
| --- | --- | --- |
| X | EP 2796101 A1 (FRAUNHOFER GES FOERDERUNG ANGEWANDTEN EV et al.), 29 October 2014 (29.10.2014), claims 1 and 5 | 1, 8, 30, 31 |
| A | CN 87100846 A (LIU, Mengtao), 24 August 1988 (24.08.1988), the whole document | 1-31 |
| A | CN 87206141 U (LIU, Yuewu et al.), 27 January 1988 (27.01.1988), the whole document | 1-31 |
| A | CN 2043493 U (LI, Zhao et al.), 30 August 1989 (30.08.1989), the whole document | 1-31 |
| A | DE 10201066 A1 (SIEMENS AG), 31 July 2003 (31.07.2003), the whole document | 1-31 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 27 December 2016 (27.12.2016) | **04 January 2017 (04.01.2017)** |

| Name and mailing address of the ISA/CN: State Intellectual Property Office of the P. R. China No. 6, Xitucheng Road, Jimenqiao Haidian District, Beijing 100088, China Facsimile No.: (86-10) 62019451 | Authorized officer **NIE, Xiaoxue** Telephone No.: (86-10) **62085018** |
| --- | --- |

Form PCT/ISA/210 (second sheet) (July 2009)

## INTERNATIONAL SEARCH REPORT
### Information on patent family members

International application No.

**PCT/CN2016/100804**

| Patent Documents referred in the Report | Publication Date | Patent Family | Publication Date |
|---|---|---|---|
| EP 2796101 A1 | 29 October 2014 | US 2016074561 A1 | 17 March 2016 |
| | | EP 2988682 A1 | 02 March 2016 |
| | | WO 2014173468 A1 | 30 October 2014 |
| | | CN 105283140 A | 27 January 2016 |
| | | JP 2016522811 A | 04 August 2016 |
| | | KR 20160004322 A | 12 January 2016 |
| | | CA 2910164 A1 | 30 October 2014 |
| | | AU 2013387207 A1 | 12 November 2015 |
| | | EP 2796101 B1 | 20 April 2016 |
| CN 87100846 A | 24 August 1988 | None | |
| CN 87206141 U | 27 January 1988 | None | |
| CN 2043493 U | 30 August 1989 | None | |
| DE 10201066 A1 | 31 July 2003 | DE 10201066 B4 | 15 July 2004 |

Form PCT/ISA/210 (patent family annex) (July 2009)

35

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009536081 A
- DE 19904569 A1
- WO 2004056275 A1
- WO 2011123274 A1
- CN 105283140 A